# EUROPEAN PATENT APPLICATION

(11) **EP 4 806 904 A1**
(43) Date of publication of application: **16.09.2026**
(21) Application number: 24888101.3
(22) Date of filing: 08.11.2024
(51) Int. Cl.: C07K 7/06, C07K 14/705, A61K 38/17, A61P 9/10, A61P 9/00, A61K 38/10

(54) **POLYPEPTIDE AND USE THEREOF**

(30) Priority: 09.11.2023 CN 202311483037
(71) Applicant: Sichuan Gooddoctor Panxi Pharmaceutical Co. Ltd, Sichuan 615000 (CN)
(72) Inventor: GENG, Yuefei, Autonomous Prefecture, Sichuan 615000 (CN)
(74) Representative: Mewburn Ellis LLP
(86) International application number: PCT/CN2024/130962
(87) International publication number: WO 2025/098490

(57) **Abstract**

The invention relates to a polypeptide and use thereof in treating stroke and other neurological injury-related diseases or use thereof in health care products.

## Description

### FIELD OF THE INVENTION

The invention relates to a polypeptide and use thereof in treating stroke and other neurological injury-related diseases or use thereof in health care products.

### BACKGROUND OF THE INVENTION

Nerve damage repair and cerebral microvascular angiogenesis are of great significance in the treatment of nervous system diseases. With an aging population and environmental changes, the incidence of neurological diseases such as stroke, Alzheimer's disease, and Parkinson's disease is rising year by year, leading to damage and death of nerve cells and seriously affecting patients' cognitive and motor functions. Since the adult brain has limited capacity for neuronal regeneration, repairing damaged neurons and rebuilding neural connections are key to improving these diseases.

The role of endogenous neurogenesis in the repair of nerve damage cannot be ignored. Studies have shown that after ischemic stroke, neural stem cells can migrate to the damaged area and differentiate into new neurons, which is a process crucial for restoring brain function. Furthermore, angiogenesis and neurogenesis mutually regulate each other, sharing some molecular mechanisms, and promoting the survival and functional recovery of nerve cells. Angiogenesis not only provides nerve cells with the necessary nutrients and oxygen, but also improves the microenvironment, reduces the formation of collagen fibers, and provides channels for new axons, thereby promoting nerve regeneration.

Good vascularization can reduce the recurrence rate of stroke and improve patients' quality of life. Therefore, treatment strategies for nerve damage should emphasize the combination of nerve damage repair and cerebral microvascular angiogenesis. Promoting endogenous neurogenesis and angiogenesis offers promising prospects for the development of novel therapeutic drugs, helping to improve cognitive and motor function in patients with neurological disorders.

### SUMMARY OF THE INVENTION

The objective of the invention is to provide a polypeptide that can be used for nerve damage repair and cerebral microvascular angiogenesis, thereby treating degenerative neurological diseases such as stroke.

In a first aspect, the invention provides a polypeptide or a physiologically compatible salt thereof, wherein the amino acid sequence of the polypeptide is selected from:
H-Ala-Leu-Pro-Ala-Pro-Gly-Thr-Leu-OH (SEQ ID No. 1);
H*-D-*Ala-Leu-Pro-Ala-Pro-Gly-Thr-Leu-OH (SEQ ID No. 2);
H-Ala*-D-*Leu-Pro-Ala-Pro-Gly-Thr-Leu-OH (SEQ ID No. 3);
H-Ala-Leu*-D-*Pro-Ala-Pro-Gly-Thr-Leu-OH (SEQ ID No. 4);
H-Ala-Leu-Pro*-D-*Ala-Pro-Gly-Thr-Leu-OH (SEQ ID No. 5);
H-Ala-Leu-Pro-Ala*-D-*Pro-Gly-Thr-Leu-OH (SEQ ID No. 6);
H-Ala-Leu-Pro-Ala-Pro-Gly*-D-*Thr-Leu-OH (SEQ ID No. 7);
H-Ala-Leu-Pro-Ala-Pro-Gly-Thr*-D-*Leu-OH (SEQ ID No. 8);
Ac-Ala-Leu-Pro-Ala-Pro-Gly-Thr-Leu-OH (SEQ ID No. 9);
H-Ala-Leu-Pro-Ala-Pro-Gly-Thr-Leu-NH2 (SEQ ID No. 10);
Ac-Ala-Leu-Pro-Ala-Pro-Gly-Thr-Leu-NH2 (SEQ ID No. 11);
H-Ala-Leu-Pro-Ala-Pro-Gly-Thr-Ala-OH (SEQ ID No. 12);
H-Ala-Leu-Ala-Ala-Pro-Gly-Thr-Leu-OH (SEQ ID No. 13);
H-Ala-Leu-Gly-Ala-Pro-Gly-Thr-Leu-OH (SEQ ID No. 14);
H-Ala-Leu-Pro-Ala-Gly-Gly-Thr-Leu-OH (SEQ ID No. 15);
H-Ala-Ala-Pro-Ala-Pro-Gly-Thr-Leu-OH (SEQ ID No. 16);
H-Ala-Leu-Pro-Ala-Ala-Gly-Thr-Leu-OH (SEQ ID No. 17);
H-Ala-Leu-Pro-Ala-Pro-Gly-Ala-Leu-OH (SEQ ID No. 18);
H-Leu-Ala-Pro-Ala-Pro-Gly-Thr-Leu-OH (SEQ ID No. 19);
H-Leu-Ala-Pro-Ala-Pro-Gly-Thr-Ile-OH (SEQ ID No. 20);
H-Ala-Leu-Pro-Ala-Pro-Gly-Thr-Ile-OH (SEQ ID No. 21);
H-Gln-Leu-Pro-Ala-Pro-Gly-Thr-Leu-OH (SEQ ID No. 22);
H-Pro-Ile-Ala-Ser-Gln-His-Leu-Asn-Leu-Ala-Pro-Ala-Pro-Gly-Thr-Ile-OH (SEQ ID No. 23);
H-Ala-Lys-Asn-Gln-Leu-Pro-Ala-Pro-Gly-Thr-Leu-Gln-His-Phe-Cys-OH (SEQ ID No. 24);
H-Gln-Leu-Pro-Ala-Pro-Gly-Thr-Leu-Gln-His-Phe-OH (SEQ ID No. 25);
H-Pro-Ala-Pro-Gly-Thr-Leu-OH (SEQ ID No. 26);
H-Pro-Ala-Pro-Gly-Thr-OH (SEQ ID No. 27);
H-Pro-Gly-Thr-Leu-OH (SEQ ID No. 28);
H-Leu-Pro-Ala-Pro-Gly-Thr-Leu-OH (SEQ ID No. 29);
H-Ala-Leu-Pro-Ala-Pro-OH (SEQ ID No. 30);
H-Leu-Pro-Ala-Pro-OH (SEQ ID No. 31);
H-Leu-Pro-Ala-Pro-Gly-Thr-OH (SEQ ID No. 32);
H-Leu-Pro-Ala-Pro-Gly-OH (SEQ ID No. 33);
H-Ala-Pro-Gly-Thr-Leu-OH (SEQ ID No. 34);
H-Ala-Leu-Pro-Ala-OH (SEQ ID No. 35);
H-Pro-Ala-Pro-Gly-OH (SEQ ID No. 36);
H-Ala-Pro-Gly-Thr-OH (SEQ ID No. 37);
H-Gly-Thr-Leu-OH (SEQ ID No. 38);
H-Ala-Leu-Pro-Ala-Pro-Gly-Thr-OH (SEQ ID No. 39);
H-Ala-Leu-Pro-Ala-Pro-Gly-Oh (SEQ ID No. 40);
Ac-Ala-Leu-Pro-Ala*-D-*Pro-Gly-Thr-Leu-NH2 (SEQ ID No. 41);
Ac-Ala-Leu-Pro*-D-*Ala-Pro-Gly-Thr-Leu-NH2 (SEQ ID No. 42).

In some embodiments, the polypeptide is selected from: compound 1, compound 2, compound 3, compound 4, compound 5, compound 6, compound 7, compound 8, compound 10, compound 11, compound 15, compound 16, compound 17, compound 18, compound 19, compound 20, compound 22, compound 23, compound 24, compound 25, compound 26, compound 27, compound 28, compound 29, compound 30, compound 31, compound 32, compound 33, compound 34, compound 35, compound 36, compound 37, compound 38, compound 39, compound 40, compound 41, compound 42, or physiologically compatible salts thereof.

In a second aspect, the invention provides the use of the polypeptide or a physiologically compatible salt thereof in the preparation of a drug for treating or preventing neurological injury-related diseases; or a method for treating or preventing neurological injury-related diseases in a subject, the method comprising administering a therapeutically effective amount of the polypeptide or physiologically compatible salt thereof to the subject; or the use of the polypeptide or physiologically compatible salt thereof for treating or preventing neurological injury-related diseases.

In some embodiments, neurological injury-related diseases involve abnormal cerebral microvascular angiogenesis.

Furthermore, the neurological injury-related disease includes stroke, diabetic neuropathy, brain injury, neurodegenerative diseases, vascular dementia, cerebrovascular diseases, and epilepsy. Furthermore, the neurodegenerative disease includes Alzheimer's disease, Parkinson's disease, amyotrophic lateral sclerosis, Huntington's disease, multiple sclerosis, and HIV-1-related dementia. Furthermore, brain injury includes chronic traumatic encephalopathy.

Furthermore, stroke includes ischemic stroke, hemorrhagic stroke, and stroke sequelae.

Furthermore, brain injury includes cerebral ischemia-reperfusion injury.

In some embodiments, the polypeptide of the invention improves neurological dysfunction. Furthermore, the polypeptide of the invention improves neurological dysfunction in stroke.

In some embodiments, the polypeptide of the invention promotes the number of hippocampal neurons, glial cells, and new blood vessels in the later stage of stroke.

In some embodiments, the polypeptide of the invention promotes neurogenesis and angiogenesis in stroke.

Furthermore, the stroke sequelae includes limb dysfunction, mental and intellectual impairment, speech dysfunction, and dysphagia. Among them, limb dysfunction includes limb paralysis, which seriously affects patients' daily living abilities; speech dysfunction includes aphasia, which affects patients' communication abilities; and mental and intellectual impairment includes depression, which places an additional burden on patients' mental health. The polypeptide of the invention can effectively improve stoke sequelae.

In a third aspect, the invention provides a composition comprising the polypeptide of the invention or a physiologically compatible salt thereof and a physiologically compatible excipient. In some embodiments, the composition of the invention is a pharmaceutical composition in any form selected from the group consisting of oral liquid, tablet, granule, capsule, dripping pill, injection, transdermal preparation, topical lotion, and implantable preparation. In some embodiments, the composition of the invention is a health care product composition.

### BRIEF DESCRIPTION OF THE DRAWINGS

FIG. 1 shows the tandem mass spectrometry (MS-MS) spectra of some polypeptides of the invention, including Figs. 1A to 1Z;
FIG. 2 illustrates the effects of the polypeptide of the invention on the proliferation and differentiation of neural stem cells at different concentrations, including Figs. 2A to 2D;
FIG. 3 illustrates the proliferation-promoting effect of the polypeptide of the invention on the neural stem cell line C17.2, including Figs. 3A to 3B;
FIG. 4 illustrates the differentiation-promoting effect of the polypeptide of the invention on primary neural stem cells (NSCs), including Figs. 4A to 4C;
FIG. 5 illustrates the angiogenesis-promoting effect of the polypeptide of the invention;
FIG. 6 shows the TTC staining results (left) and the statistical graph of cerebral infarction volume (right) for the effect of the polypeptide of the invention on the cerebral infarction volume of rats with stroke;
FIG. 7 shows the HE staining results for the effect of the polypeptide of the invention on pathological damage of hippocampal and cortical cells in rats with stroke;
FIG. 8 is a statistical chart of the mNSS score for the effect of the polypeptide of the invention on the neurological function of rats with stroke;
FIG. 9 shows the statistical results of the rotarod test for the effect of the polypeptide of the invention on the balance ability and endurance of rats with stroke;
FIG. 10 shows the immunofluorescence staining results for the effect of the polypeptide of the invention on the proliferation of hippocampal cells in rats with stroke;
FIG. 11 shows the immunofluorescence staining results for the effect of the polypeptide of the invention on the differentiation of neural stem cells in the hippocampus of rats with stroke (DCX and BrdU immunofluorescence double staining image (left), GFAP and BrdU immunofluorescence double staining image (right));
FIG. 12 shows the immunofluorescence staining for the effect of the polypeptide of the invention on the expression of the vascular marker protein CD31 in the hippocampus and cortex of rats with stroke;
FIG. 13 shows how the polypeptide of the invention improves pathological tissue damage and alleviates cortical tissue atrophy in rats; and
FIG. 14 shows the effect of the polypeptide of the invention on the expression of PSD95 and SYP in the hippocampus and cortex of rats.

### DETAILED DESCRIPTION OF THE INVENTION

The invention is illustrated by the following specific examples, which are for exemplary purposes only and do not limit the scope of protection of the invention in any way.

As used in the present application (including the attached claims), unless the context otherwise clearly indicates, singular forms of words such as "a/an" and "the" include their corresponding plural referents.

Unless otherwise clearly indicated by the context, the term "or" is used to refer to the term "and/or" and may be used interchangeably with the term "and/or".

The term "effective amount" or "therapeutically effective amount" refers to the amount of an active ingredient (such as a compound) which when administered to a subject to treat a disease, or at least one clinical symptom of a disease or disorder, is sufficient to influence the treatment of the disease, disorder or symptom. The "therapeutically effective amount" may vary depending on the compound, the disease, disorder and/or symptoms of the disease or disorder, the severity of the disease, disorder and/or symptoms of the disease or disorder, the age of the subject to be treated and/or the weight of the subject to be treated. In any given case, the appropriate amount will be apparent to those skilled in the art, or can be determined by routine experimentation. In some embodiments, a "therapeutically effective amount" is an amount of at least one compound and/or at least one stereoisomer thereof, and/or at least one pharmaceutically acceptable salt thereof, disclosed in the present application, that is effective for the "treatment" (as defined above) of a disease or disorder in a subject. In the case of a combination therapy, "therapeutically effective amount" is the total amount of the combined objects used to effectively treat a disease, disorder or condition.

The term "physiologically compatible salt" refers to a salt form which is physiologically compatible (i.e., pharmacologically acceptable), and is substantially non-toxic to the individual to which the compounds of the invention will be administered. Physiologically compatible salts of the compounds of the invention include conventional and stoichiometric acid addition salts or base addition salts formed from suitable non-toxic organic or inorganic acids or inorganic bases. In some embodiments, physiologically compatible salts include hydrochloride, trifluoromethanesulfonate, etc.

The term "physiologically compatible excipient" refers to any component that is compatible with the environment in a living organism and does not cause adverse reactions (e.g., a vehicle that enables the polypeptides of the invention to suspend, form complexes or dissolve). Excipients include, but are not limited to, anti-adhesives, antioxidants, binders, coatings, compression aids, disintegrants, pigments, softeners, emulsifiers, fillers, film-forming or coating agents, flavoring agents, fragrances, glidants, lubricants, preservatives, printing inks, adsorbents, suspending or dispersing agents, sweeteners, water, and the like.

The term "pharmaceutical composition" refers to a preparation comprising the polypeptide of the invention and a physiologically compatible excipient, with the purpose of promoting the therapeutic or preventive effects of the polypeptide in vivo to alleviate the corresponding symptoms. The composition of the invention may be in any form suitable for any route of administration or conventional use.

The term "health care product composition" refers to any composition that includes, in addition to physiologically compatible excipients, food ingredients such as macronutrients, micronutrients, plants or plant extracts, or substances with nutritional or physiological effects, with the aim of supplementing a human diet to improve the latter's nutritional status and thereby promote good health.

The term "subject" includes mammals such as humans, rats, mice, dogs, and monkeys, but is usually a human.

The term "neurological injury-related disease" includes a variety of conditions such as stroke, diabetic neuropathy, brain injury, neurodegenerative diseases, and epilepsy, with stroke being the most common and serious.

Stroke is an acute neurological dysfunction caused by cerebrovascular problems, and it is usually divided into ischemic stroke and hemorrhagic stroke. Ischemic stroke accounts for 87% of all cases, mainly due to reduced blood flow caused by vascular occlusion or stenosis; while hemorrhagic stroke is caused by bleeding due to rupture of blood vessels[3].

Diabetic neuropathy is a common complication of diabetes, characterized by nerve damage that leads to sensory loss, pain, and motor dysfunction.

Brain injury can be traumatic (such as traumatic brain injury) or acquired (such as brain injury caused by stroke, tumor, etc.). Brain injury can lead to loss of consciousness, altered thinking, and motor problems; the specific symptoms depend on the location and extent of the injury.

Neurodegenerative diseases are a class of diseases characterized by the gradual loss of function and death of nerve cells. These diseases typically affect motor, cognitive, and autonomic nervous system functions, leading to a significant decline in patients' quality of life. The neurodegenerative disease generally includes Alzheimer's disease, Parkinson's disease, amyotrophic lateral sclerosis, Huntington's disease, multiple sclerosis, and HIV-1-related dementia, etc.

Epilepsy is a common neurological disorder characterized by abnormal electrical discharges in the brain, leading to recurrent epileptic seizures. Epilepsy can manifest in various forms and may affect patient's consciousness, movement, sensation, and behavior.

The abbreviations for amino acid used in the present application are as follows.

**Table 1 English names or abbreviations and corresponding Chinese names of reagents and solvents used in the description**

| Chinese name | English name | Abbreviation |
|---|---|---|
| Alanine | Alanine | Ala |
| Arginine | Arginine | Arg |
| Asparagine | Asparagine | Asn |
| Aspartic acid | Aspartic acid | Asp |
| Cysteine | Cysteine | Cys |
| Glutamine | Glutamine | Gln |
| Glutamic acid | Glutamic acid | Glu |
| Glycine | Glycine | Gly |
| Histidine | Histidine | His |
| Isoleucine | Isoleucine | Ile |
| Leucine | Leucine | Leu |
| Lysine | Lysine | Lys |
| Methionine | Methionine | Met |
| Phenylalanine | Phenylalanine | Phe |
| Proline | Proline | Pro |
| Serine | Serine | Ser |
| Threonine | Threonine | Thr |
| Tryptophan | Tryptophan | Trp |
| Tyrosine | Tyrosine | Tyr |
| Valine | Valine | Val |

| **English name and abbreviation** | **Chinese name** |
|---|---|
| Ac | Acetyl |
| 2-Cl-CTC | 2-chlorotrityl chloride Resin |
| DMF | *N, N*-dimethyl formamide |
| Rink Amide Resin | Rink Amide Resin |
| Fmoc | 9-fluorenylmethoxycarbonyl |
| DCM | Dichloromethane |
| PIP | Piperidine |
| HOBt | 1-hydroxybenzotriazole |
| DIEA | N,N-diisopropylethylamine |
| MeOH | Methanol |
| TFA | Trifluoroacetic acid |
| TIS | Triisopropylsilane |
| | *N,N'*-diisopropyl |
| DIC | carbodiimide |

### Example 1: Chemical synthesis of polypeptides

The polypeptides of the invention were synthesized using the conventional Fmoc solid-phase synthesis method. Polypeptides with a carboxyl group at the C-terminus were synthesized using 2-chlorotriphenylmethyl chloride resin, while polypeptides with an amide group at the C-terminus were synthesized using Rink amide resin.

Generally, it comprises three steps: 1) preparation of fully protected peptide resin, which involves sequentially linking amino acids, including D-amino acids according to the polypeptide sequence (for N-terminally acetylated polypeptides, acetylation is performed after completion of polypeptide sequence linking and deprotection of Fmoc); 2) cleavage, which involves cleaving the polypeptide from the resin while removing the amino acid side chain protecting groups; 3) purification by reversed-phase preparative high-performance liquid chromatography (HPLC) and lyophilization.

### Step 1: Preparation of fully protected peptide resin

### 1.1 Preparation of peptide resins containing polypeptides with a carboxyl group at the C-terminus

The linking procedures for the peptide sequence in the carboxy peptide resin are shown in Table 2:

**Table 2 Peptide sequence linking**

| **Step** | **Operation** | **Comment** |
|---|---|---|
| Resin linking | 1. Weigh an appropriate amount of 2-Cl-CTC resin and transfer it to a synthesis tube with DMF. | First amino acid |
| | 2. Drain the solvent, add 50 mL of DMF, and mix for 5-15 min. | |
| | 3. Dissolve the amino acid in 20 mL of DMF, cool, add 20 mL of DIEA (2.0 eq)/DMF solution, and activate for 10-15 min. | |
| | 4. Drain the solvent, add the above activation solution, and react for 60 min. | |
| | 5. Drain the solvent, add 20-40 mL of blocking solution, DMF:MeOH:DIEA = 80:15:5 (v/v/v), and react for 30 min. | |
| | 6. Drain the solvent and wash 3 times with 40-50 mL of DMF, 1 min each time. | |
| Deprotection | 1. Drain the solvent, add 40-50 mL of 20% PIP/DMF solution, and react for 15 min. | |
| | 2. Drain the solvent, wash 6 times with 40-50 mL of DMF each time, 1 min each time, and take the resin for testing on the 4th time. | |
| Peptide linking | 1. Dissolve the amino acid in 20 mL of DMF, cool, sequentially add 10 mL of HOBt (2.33 eq)/DMF solution and 10 mL of DIC (2.67 eq)/DMF solution, and activate for 20-30 min. | This operation is performed repeatedly based on the amino acid sequence |
| | 2. Drain the solvent, add the above activation solution, react for 60 min, drain the solvent, and take the resin for testing. | |
| | 3. Wash 3 times with 40-50 mL of DMF each time, 1 min each time. | |
| Deprotection | 1. Drain the solvent, add 40-50 mL of 20% PIP/DMF solution, and react for 15 min. | |
| | 2. Drain the solvent, wash 6 times with 40-50 mL of DMF each time, 1 min each time, and take the resin for testing on the 4th time. | |
| | 3. Proceed to the acetylation process or wash 6 times with 50 mL of DCM, then allow the peptide resin to air dry naturally. | |

### 1.2 Preparation of peptide resins containing polypeptides with amide at the C-terminus

The linking procedures for the peptide sequence in the amide peptide resin are shown in Table 3:

**Table 3 Peptide sequence linking**

| **Step** | **Operation** | **Comment** |
|---|---|---|
| Resin linking | 1. Weigh an appropriate amount of Rink amide resin and transfer it to a synthesis tube with DMF. | |
| | 2. Drain the solvent, add 50 mL of DMF, and mix for 5 min. | |
| | 3. Drain the solvent, add 40 mL of 20% Pip/DMF solution, and react for 15 min. | |
| | 4. Drain the solvent and wash 5 times with 40 mL of DMF, 1 min each time. | |
| Peptide linking | 1. Dissolve the amino acid in 20 mL of DMF, cool, sequentially add 10 mL of HOBt (2.33 eq)/DMF solution and 10 mL of DIC (2.67 eq)/DMF solution, and activate for 20-30 min. | This operation is performed repeatedly based on the amino acid sequence |
| | 2. Drain the solvent, add the above activation solution, react for 60 min, drain the solvent and take the resin for testing. | |
| | 3. Wash 3 times with 40-50 mL of DMF each time, 1 min each time. | |
| Deprotection | 1. Drain the solvent, add 40-50 mL of 20% PIP/DMF solution, and react for 15 min. | |
| | 2. Drain the solvent, wash 6 times with 40-50 mL of DMF each time, 1 min each time, and take the resin for testing on the 4th time. | |
| | 3. Proceed to the acetylation process or wash 6 times with 50 mL of DCM, then allow the peptide resin to air dry naturally. | |

Acetylation: The peptide resin in the synthesis tube was flushed out with DMF, filtered under reduced pressure, and the resin was collected. 40 mL of DMF:acetic anhydride:DIEA = 85:10:5 (v/v/v) was added, and reaction was allowed with nitrogen bubbling for 30 min. After the reaction was confirmed complete, the peptide resin was washed with 100 mL of DMF each time for 3 times, 1-2 min per wash. The peptide resin was washed 5 times with 100 mL of DCM each time, for 1-2 min per wash, and the peptide resin was air dried naturally.

### Step 2: Cleaving

To the naturally air-dried peptide resin, 50-100 mL of TFA:TIS:H₂O = 94:3:3(v/v/v) was added, and the mixture was bubbled with nitrogen, and reacted for 1-2 h. The liquid was collected by suction filtration. The resin was washed once with 10-20 mL of TFA, the filtrate was combined, and concentrated under reduced pressure at 40°C until the volume was reduced to 1/3 of the original volume. 200 mL of pre-cooled isopropyl ether was added, and the mixture was stirred to precipitate, filtered, and the solid was washed 3 times with 30 mL of isopropyl ether per wash. The white solid was placed in a vacuum drying oven and dried under vacuum at 40°C and -0.08 MPa to give the crude product, which was then analyzed by LC-MS.

### Step 3. Purification by reversed-phase preparative liquid chromatography and lyophilization

Purification by reversed-phase preparative liquid chromatography (HPLC) is divided into two stages: one is purification and separation for the purpose of removing impurities, and the other is purification for the purpose of salt conversion (i.e., replacement with acetate). Generally speaking, when the purity of the crude product is greater than 90%, direct one-step purification with salt conversion can be adopted.

### (1) Preparation and purification of polypeptides by HPLC

### A. Chromatographic parameters

Column: YMC-Actus Triart C18, 30*250mm
Eluent A: 10 mM aqueous ammonium bicarbonate solution
Eluent B: acetonitrile
Flow rate: 25ml/min
UV detection wavelength: 220nm

### B. Operating steps

a) Dissolving the crude peptide in water and/or acetonitrile, and filtering through a 0.45 µm filter membrane
b) Sample injection
c) Gradient elution with eluents A and B
d) Collecting the target peptide eluate
e) Rotary evaporation for concentration

### (2) Polypeptide salt conversion (acetate) by HPLC

### A. Chromatographic parameters

Column: YMC-Actus Triart C18, 30*250mm
Eluent A: 0.1% acetic acid
Eluent B: acetonitrile
Flow rate: 25ml/min
UV detection wavelength: 220nm

### B. Operating steps

a) Equilibrating the chromatographic column with 95% A + 5% B
b) Sample injection
c) Gradient elution with eluents A and B
d) Collecting the target peptide eluate
e) Rotary evaporation for concentration

The concentrate was freeze-dried to give the target polypeptide in pure form. The sample was weighed and sent for purity, moisture and acetic acid content testing. Then, the structure was confirmed by high-resolution mass spectrometry (HRMS) and tandem mass spectrometry (MS-MS), and the results are shown in Table 4.

**Table 4. Structural sequences and precise molecular weights of the polypeptides synthesized in the invention**

| **Name** | **SEQ ID No.** | **Sequence** | **Precise molecular weight** | **Deviation ppm** |
|---|---|---|---|---|
| Compound 1 | 1 | H-Ala-Leu-Pro-Ala-Pro-Gly-Thr-Leu- OH | 738.4258 | -2.4 |
| Compound 2 | 2 | H-*D*-Ala-Leu-Pro-Ala-Pro-Gly-Thr- Leu-OH | 738.4262 | -1.9 |
| Compound 3 | 3 | H-Ala-*D*-Leu-Pro-Ala-Pro-Gly-Thr- Leu-OH | 738.4263 | -1.8 |
| Compound 4 | 4 | H-Ala-Leu-*D*-Pro-Ala-Pro-Gly-Thr- Leu-OH | 738.4263 | -1.8 |
| Compound 5 | 5 | H-Ala-Leu-Pro-*D*-Ala-Pro-Gly-Thr- Leu-OH | 738.4268 | -1.1 |
| Compound 6 | 6 | H-Ala-Leu-Pro-Ala-D-Pro-Gly-Thr- Leu-OH | 738.4263 | -1.8 |
| Compound 7 | 7 | H-Ala-Leu-Pro-Ala-Pro-Gly-D-Thr- Leu-OH | 738.4263 | -1.8 |
| Compound 8 | 8 | H-Ala-Leu-Pro-Ala-Pro-Gly-Thr-*D*- Leu-OH | 738.4263 | -1.8 |
| Compound 9 | 9 | Ac-Ala-Leu-Pro-Ala-Pro-Gly-Thr-Leu- OH | 780.4367 | -1.9 |
| Compound 10 | 10 | H-Ala-Leu-Pro-Ala-Pro-Gly-Thr-Leu- NH2 | 737.4423 | -1.8 |
| Compound 11 | 11 | Ac-Ala-Leu-Pro-Ala-Pro-Gly-Thr-Leu- NH2 | 779.4531 | -1.3 |
| Compound 12 | 12 | H-Ala-Leu-Pro-Ala-Pro-Gly-Thr-Ala- OH | 696.3792 | -2.0 |
| Compound 13 | 13 | H-Ala-Leu-Ala-Ala-Pro-Gly-Thr-Leu- OH | 712.4105 | -2.0 |
| Compound 14 | 14 | H-Ala-Leu-Gly-Ala-Pro-Gly-Thr-Leu- OH | 698.3948 | -2.1 |
| Compound 15 | 15 | H-Ala-Leu-Pro-Ala-Gly-Gly-Thr-Leu- OH | 698.3948 | -2.1 |
| Compound 16 | 16 | H-Ala-Ala-Pro-Ala-Pro-Gly-Thr-Leu- OH | 696.3792 | -2.0 |
| Compound 17 | 17 | H-Ala-Leu-Pro-Ala-Ala-Gly-Thr-Leu- OH | 712.4104 | -2.1 |
| Compound 18 | 18 | H-Ala-Leu-Pro-Ala-Pro-Gly-Ala-Leu- OH | 708.4155 | -2.1 |
| Compound 19 | 19 | H-Leu-Ala-Pro-Ala-Pro-Gly-Thr-Leu- OH | 738.4261 | -2.0 |
| Compound 20 | 20 | H-Leu-Ala-Pro-Ala-Pro-Gly-Thr-Ile- OH | 738.4262 | -1.9 |
| Compound 21 | 21 | H-Ala-Leu-Pro-Ala-Pro-Gly-Thr-Ile- OH | 738.4262 | -1.9 |
| Compound 22 | 22 | H-Gln-Leu-Pro-Ala-Pro-Gly-Thr-Leu- OH | 795.4477 | -1.8 |
| Compound 23 | 23 | H-Pro-Ile-Ala-Ser-Gln-His-Leu-Asn- Leu-Ala-Pro-Ala-Pro-Gly-Thr-Ile-OH | 1598.8758 | -1.4 |
| Compound 24 | 24 | H-Ala-Lys-Asn-Gln-Leu-Pro-Ala-Pro- Gly-Thr-Leu-Gln-His-Phe-Cys-OH | 1623.8175 | -1.0 |
| Compound 25 | 25 | H-Gln-Leu-Pro-Ala-Pro-Gly-Thr-Leu- Gln-His-Phe-OH | 1207.6330 | -1.7 |
| Compound 26 | 26 | H-Pro-Ala-Pro-Gly-Thr-Leu-OH | 554.3051 | -2.3 |
| Compound 27 | 27 | H-Pro-Ala-Pro-Gly-Thr-OH | 441.2213 | -2.3 |
| Compound 28 | 28 | H-Pro-Gly-Thr-Leu-OH | 386.2155 | -2.6 |
| Compound 29 | 29 | H-Leu-Pro-Ala-Pro-Gly-Thr-Leu-OH | 667.3891 | -2.1 |
| Compound 30 | 30 | H-Ala-Leu-Pro-Ala-Pro-OH | 467.2733 | -2.4 |
| Compound 31 | 31 | H-Leu-Pro-Ala-Pro-OH | 396.2363 | -2.5 |
| Compound 32 | 32 | H-Leu-Pro-Ala-Pro-Gly-Thr-OH | 554.3052 | -2.2 |
| Compound 33 | 33 | H-Leu-Pro-Ala-Pro-Gly-OH | 453.2577 | -2.2 |
| Compound 34 | 34 | H-Ala-Pro-Gly-Thr-Leu-OH | 457.2525 | -2.4 |
| Compound 35 | 35 | H-Ala-Leu-Pro-Ala-OH | 370.2208 | -2.2 |
| Compound 36 | 36 | H-Pro-Ala-Pro-Gly-OH | 340.1738 | -2.6 |
| Compound 37 | 37 | H-Ala-Pro-Gly-Thr-OH | 344.1687 | -2.6 |
| Compound 38 | 38 | H-Gly-Thr-Leu-OH | 289.1630 | -2.8 |
| Compound 39 | 39 | H-Ala-Leu-Pro-Ala-Pro-Gly-Thr-OH | 625.3422 | -2.1 |
| Compound 40 | 40 | H-Ala-Leu-Pro-Ala-Pro-Gly-Oh | 524.2947 | -2.1 |
| Compound 41 | 41 | Ac-Ala-Leu-Pro-Ala-D-Pro-Gly-Thr- Leu-NH2 | 779.4525 | -2.1 |
| Compound 42 | 42 | Ac-Ala-Leu-Pro-D-Ala-Pro-Gly-Thr- Leu-NH2 | 779.4526 | -1.9 |

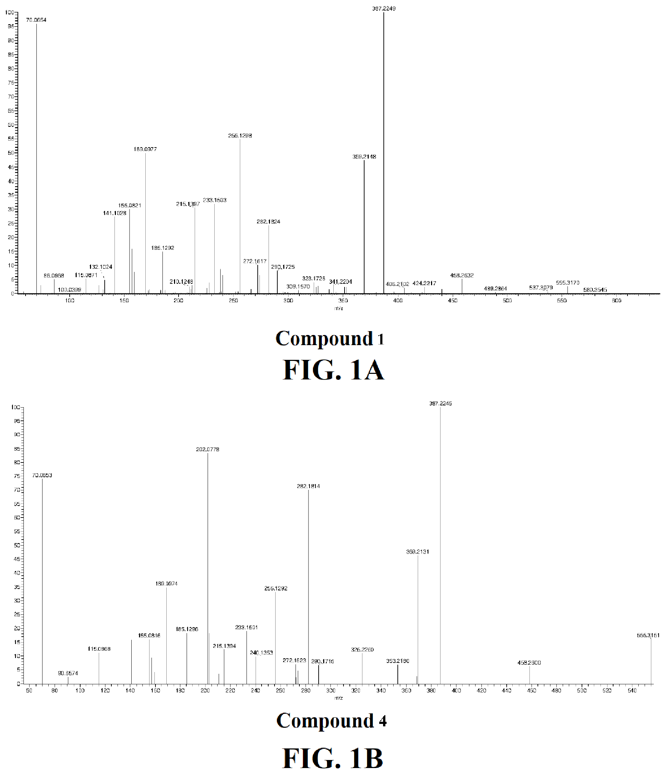

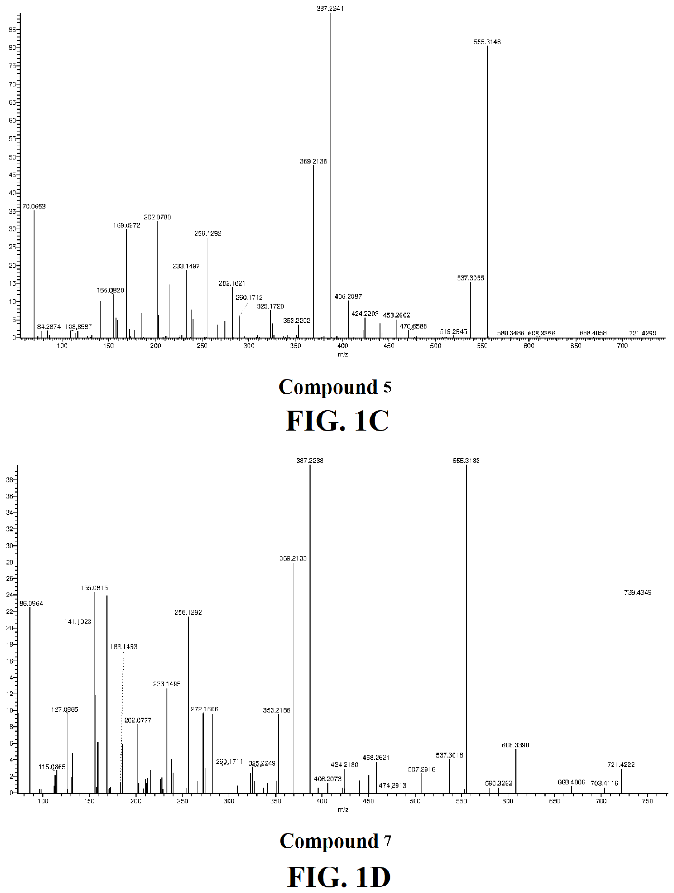

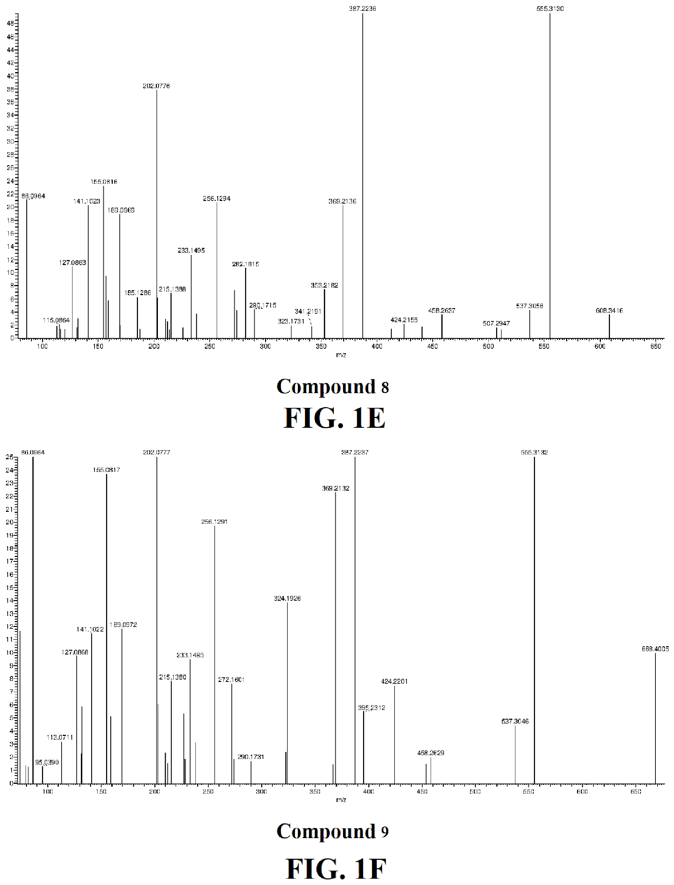

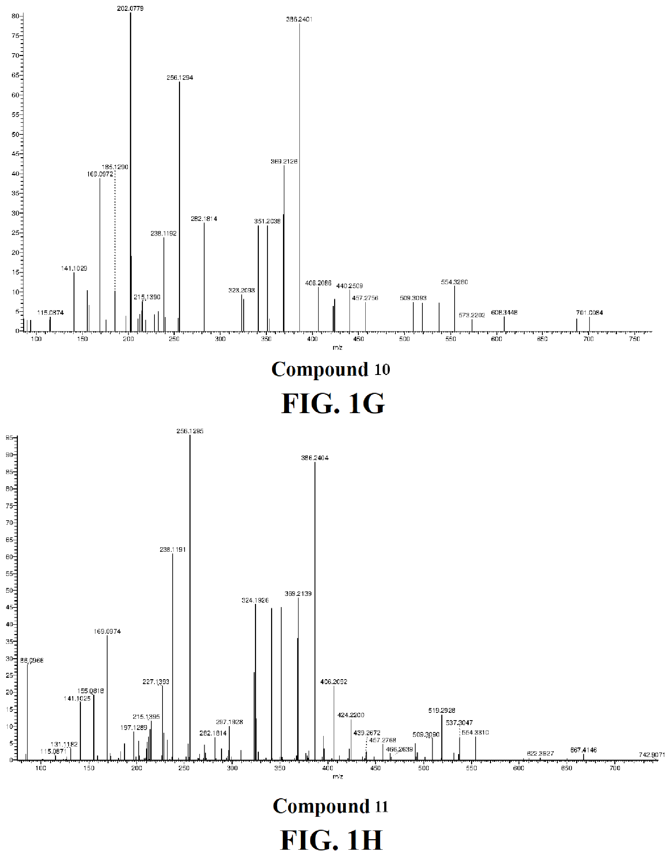

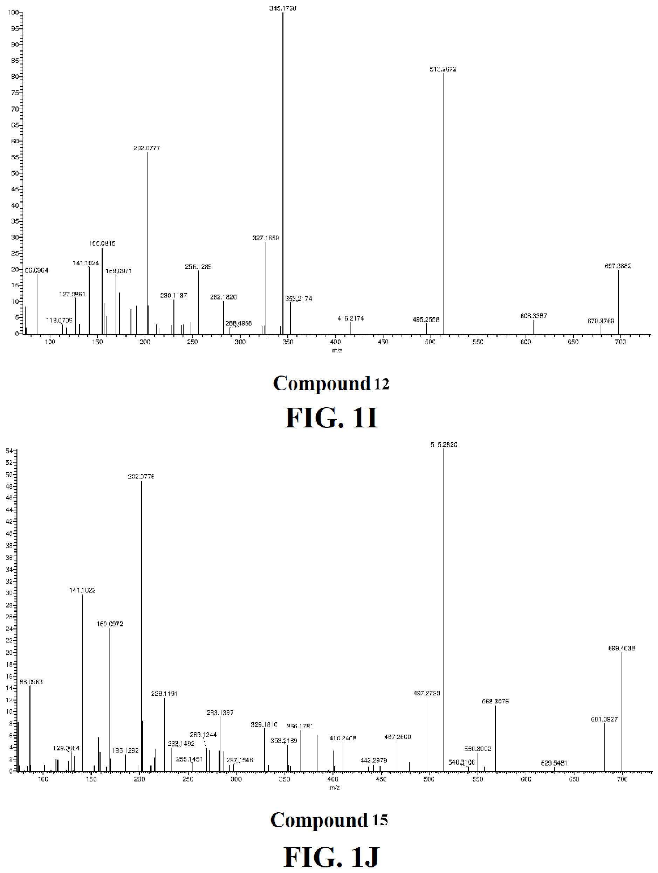

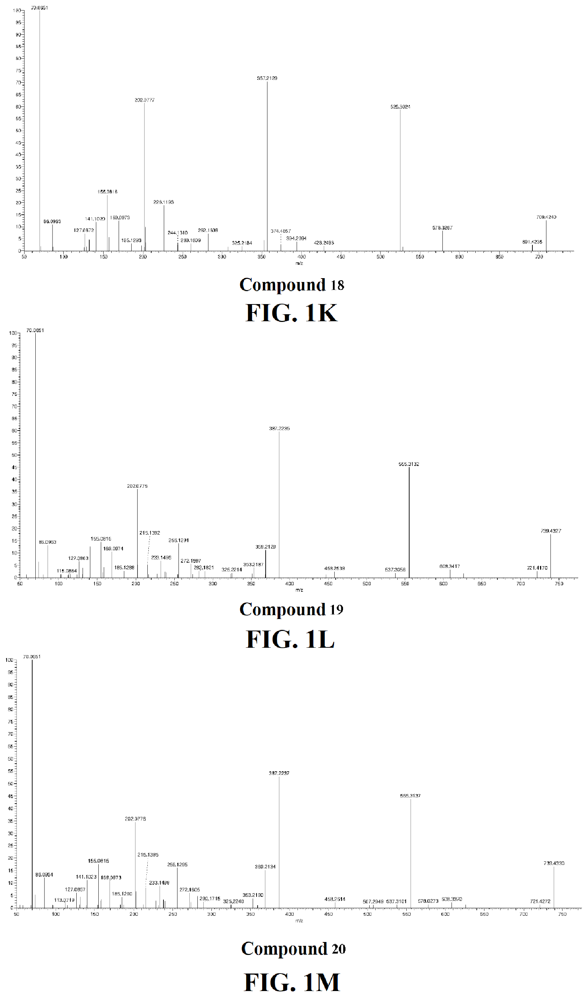

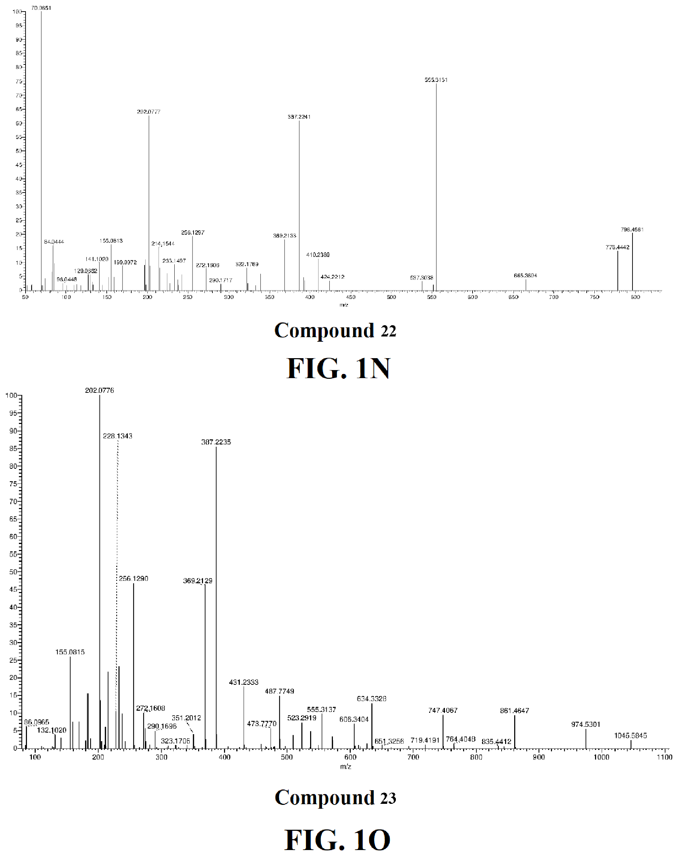

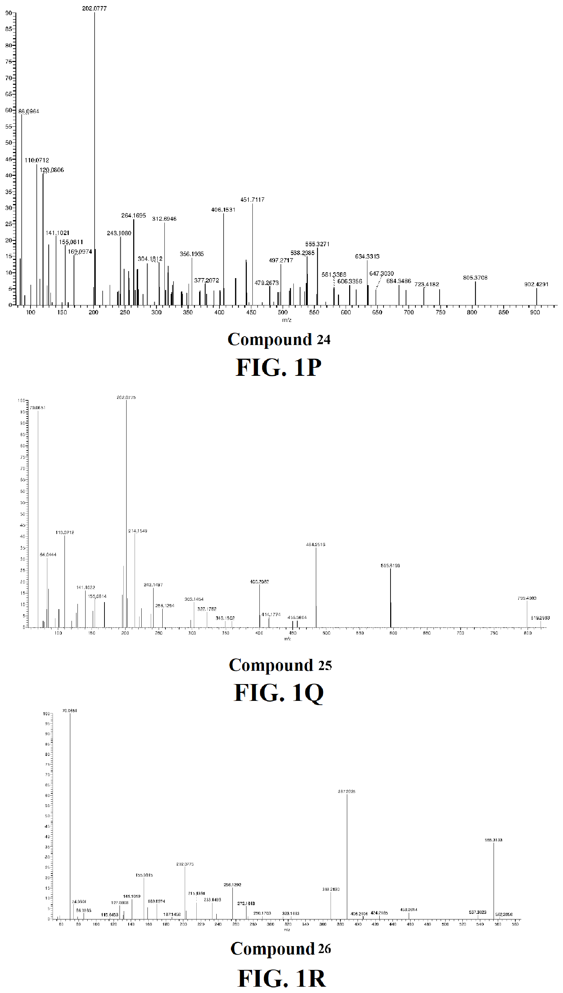

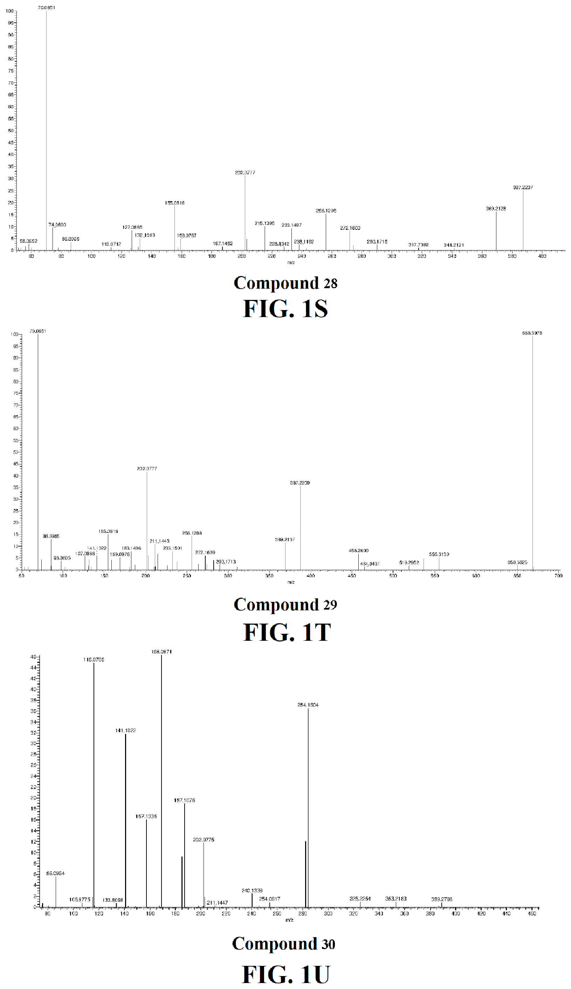

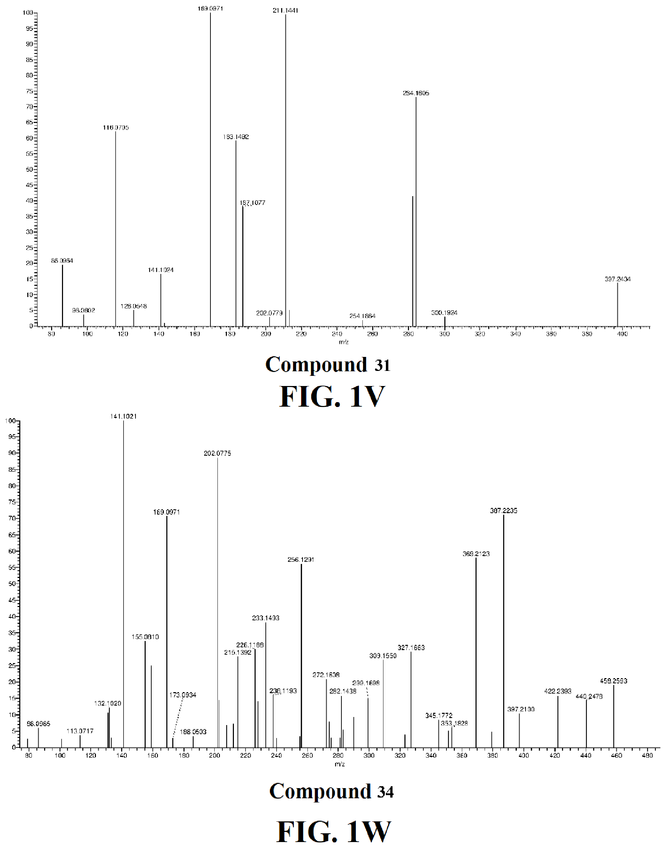

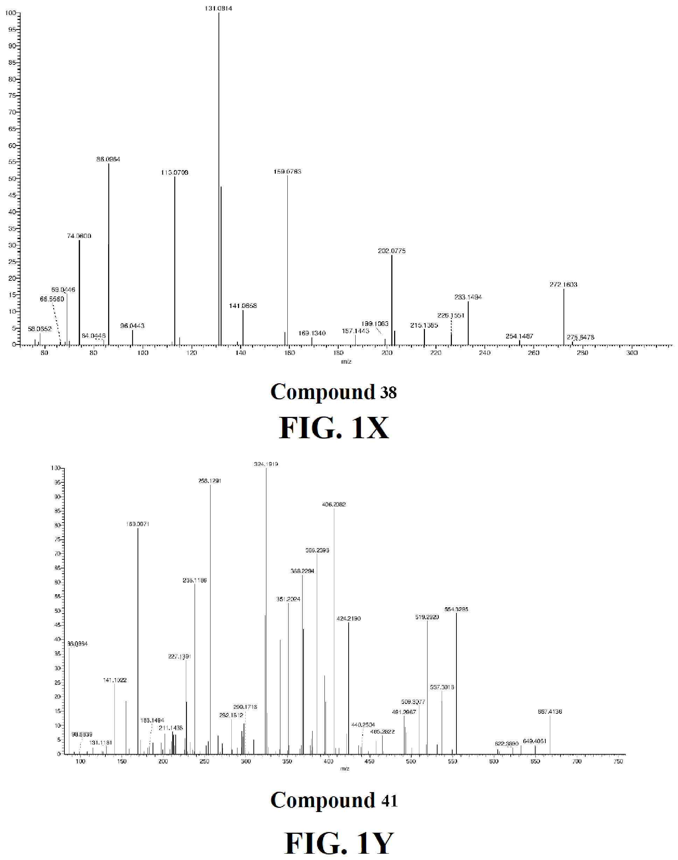

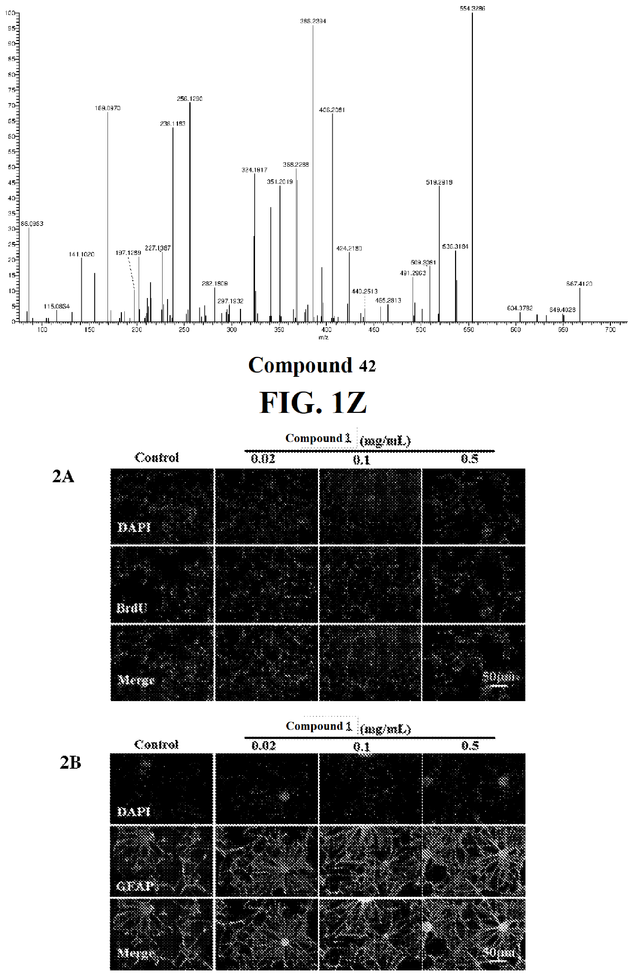

The deviation of the precise molecular weight of all the polypeptides of the invention determined by high-resolution mass spectrometry (HRMS) in the table above is ≤-2.8ppm, which is consistent with the theoretical molecular weight of the corresponding polypeptides of the invention. The fragment ions of the polypeptide samples analyzed by tandem mass spectrometry (MS-MS) have a high degree of matching with the theory (see Fig. 1), which can confirm that their polypeptide sequences are consistent with the corresponding polypeptide structures of the invention.

### Example 2: Study on the proliferation and differentiation of neural stem cells promoted by the polypeptide of the invention at different concentrations

This part of the experiment requires feeding male and female rats in advance, then extracting hippocampal neural stem cells from newborn SD rats within 24 h of birth, culturing them to P3 for identification, and then using them for experiments.

Extraction and culture of primary neural stem cells (NSCs): Hippocampal neural stem cells were extracted from neonatal SD mice 24 h after birth. The procedure is as follows: first, the neonatal rats were disinfected by soaking in 75% ethanol, then transferred to a biological safety cabinet for manipulation under aseptic conditions. After the entire brain was removed from the neonatal mouse, it was immediately placed in cold D-Hanks solution to remove non-neural tissue. Under a dissecting microscope, the meninges were carefully removed and the hippocampal structures on both sides were separated. The tissue was mechanically broken into flocculent pieces with a scalpel, pipetted, and the tissue sample was transferred to a 15 mL centrifuge tube and centrifuged at 800 rpm for 5 min. After centrifugation, the supernatant was discarded, and Accutas digestion enzyme was added at a ratio of 100 µL per neonatal mouse brain. The tissues were incubated in a 37°C incubator for 5 min to facilitate digestion. Digestion was then terminated by adding complete DMEM/F12 medium. The cell suspension was obtained by filtering through a 200-mesh sieve, followed by centrifugation at 800 rpm for 5 min. The supernatant was discarded, complete DMEM/F12 medium was added, and the cells were gently resuspended by pipetting. The cells were seeded into T25 flasks. The flasks were cultured in an incubator with 5% CO2 at 37 °C. The flasks were not disturbed for the first 3 days. Subsequently, half-medium changes were performed every 2 to 3 days, and subculture was conducted every 6 to 7 days.

### 2.1 Evaluation of the proliferation-promoting activity of the polypeptides of the invention using primary neural stem cells (NSCs).

Medium-sized neurospheres formed from P3-P4 generations were centrifuged at 800 rpm for 5 min, the supernatant was discarded, a small amount of complete medium was added and pipetted to form a single-cell suspension, the cells were resuspended, counted, and seeded at 1.0 x 105 cells/mL into 24-well plates coated with 25 µg/mL PLL. The cells were treated with different concentrations of peptide compound 1, with the final concentrations of the peptide in the invention being (0.02, 0.1, 0.5 mg/mL). The cells were then incubated for 48 h. After 44 h of treatment, BrdU was incorporated to a final concentration of 10 µM. Immunofluorescence staining was performed 4 h later, and the cells were then observed and photographed under a fluorescence microscope. The BrdU-positive cell rate was statistically analyzed.

### 2.2 Evaluation of the differentiation-promoting activity of the polypeptides of the invention using primary neural stem cells (NSCs).

Medium-sized neurospheres cultured at P3-P4 were collected, centrifuged at 800 rpm for 5 min, and the supernatant was discarded. A small amount of differentiation medium (DMEM/F12 differentiation medium containing 2% fetal bovine serum) was added, and the mixture was pipetted to obtain a single-cell suspension. After resuspension and cell counting, the cells were seeded at 2.0×105 cells/mL in 24-well plates coated with 25 µg/mL PLL. After overnight incubation for cell adherence, the cells were treated with polypeptide compound 1 at different concentrations. The final concentrations of the polypeptide of the present invention were 0.02, 0.1, and 0.5 mg/mL, and differentiation induction was continued for 4 days. Fluorescent staining was performed after differentiation was completed. Primary antibodies MAP-2 (Rabbit, 1:200), GFAP (Mouse, 1:200); secondary antibodies Cy3-labeled goat anti-rabbit (1:200), FITC-labeled goat anti-mouse (1:200). Subsequently, the images were observed and collected under a fluorescence microscope, and the mean fluorescence intensities of MAP-2 and GFAP were statistically analyzed respectively.

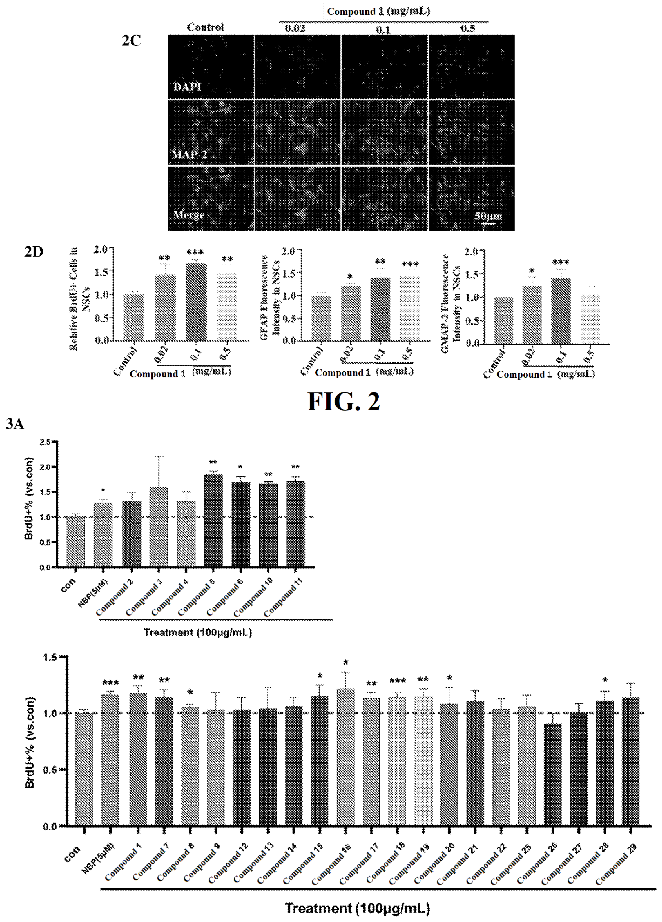

Experimental results: As shown in Fig. 2, compared with the control group, all tested concentrations of polypeptide compound 1 can promote the proliferation and differentiation of primary neural stem cells (NSCs). Specifically, the percentage of BrdU-positive cells was significantly increased, and the mean fluorescence intensities of both MAP-2 and GFAP were significantly elevated. Overall, 0.1 mg/mL (100 µg/mL) exhibited a better effect.

### Example 3: Study on the proliferation and differentiation of neural stem cells promoted by the polypeptide of the invention

### 1.1 Evaluation of the proliferation-promoting activity of the polypeptides of the invention using the neural stem cell line C17.2.

(1) C17.2 cells were seeded in 96-well plates at a concentration of 1500 cells/100 µL/well. After overnight culture for cell adherence, the cells were treated with drugs;
(2) Drug treatment was applied. The final concentration of the polypeptide of the invention was 100 µg/mL. Meanwhile, butylphthalide (NBP, 5 µM) was set as the positive control. The cells were cultured in the incubator for another 48 h;
(3) BrdU was added at 44 h of treatment to a final concentration of 10 µM. Immunofluorescence staining was performed 4 h later, and the percentage of BrdU-positive cells was statistically analyzed using a high-content cell imaging analysis system.

### 1.2 Evaluation of the differentiation-promoting activity of the polypeptides of the invention using primary neural stem cells (NSCs).

This part of the experiment requires feeding male and female rats in advance, then extracting hippocampal neural stem cells from newborn SD rats within 24 h of birth, culturing them to P3 for identification, and then using them for experiments.
(1) Extraction and culture of primary neural stem cells (NSCs)
(2) NSCs that have been passaged to P3 and have good viability were seeded in differentiation medium (2% FBS+DMEM/F12 1:1 medium) in 48-well plates with a cell concentration of 5×104 cells/mL per well. After overnight culture for cell adherence, the cells were treated with drugs;
(3) Drug treatment was applied. The final concentration of the polypeptide of the invention was 100 µg/mL. Meanwhile, nerve growth factor (NGF, 50 ng/mL) was set as the positive control, and the cells were cultured in the incubator for another 4 days;
(4) Immunofluorescence staining (primary antibody βIII-tubulin) was performed after 4 days of treatment. The average fluorescence intensity of βIII-tubulin was analyzed and the Neurite Outgrowth Assay was performed using a high-content cell imaging analysis system.

### Experimental result 1: The proliferation-promoting effect of the polypeptide of the invention on the neural stem cell line C17.2 cells.

The effect of the polypeptide of the present invention on the proliferation of C17.2 cells was analyzed and quantified using a high-content cell imaging analysis system. The results are shown in Table 5:

**Table 5 The proliferation-promoting results of the polypeptide of the invention on the neural stem cell line C17.2 cells**

| **The polypeptide of the invention** | **Proliferation-promoting results** | **The polypeptide of the invention** | **Proliferation-promoting results** | **The polypeptide of the invention** | **Proliferation-promoting results** |
|---|---|---|---|---|---|
| Compound 2 | / | Compound 16 | * | Compound 30 | / |
| Compound 3 | / | Compound 17 | ** | Compound 31 | / |
| Compound 4 | / | Compound 18 | *** | Compound 32 | * |
| Compound 5 | ** | Compound 19 | ** | Compound 33 | / |
| Compound 6 | * | Compound 20 | * | Compound 34 | / |
| Compound 7 | ** | Compound 21 | / | Compound 35 | / |
| Compound 8 | * | Compound 22 | / | Compound 36 | ** |
| Compound 9 | / | Compound 23 | / | Compound 37 | * |
| Compound 10 | ** | Compound 24 | / | Compound 38 | / |
| Compound 11 | ** | Compound 25 | / | Compound 39 | / |
| Compound 12 | / | Compound 26 | / | Compound 40 | * |
| Compound 13 | / | Compound 27 | / | Compound 41 | / |
| Compound 14 | / | Compound 28 | * | Compound 42 | / |
| Compound 15 | * | Compound 29 | / | | |

Compared with the blank control group, *p < 0.05, **p < 0.01, ***p < 0.001; significant differences were observed in all 3 batches of experiments using the positive drug NBP; "/" indicates no significant difference, and some samples showed cytotoxicity.

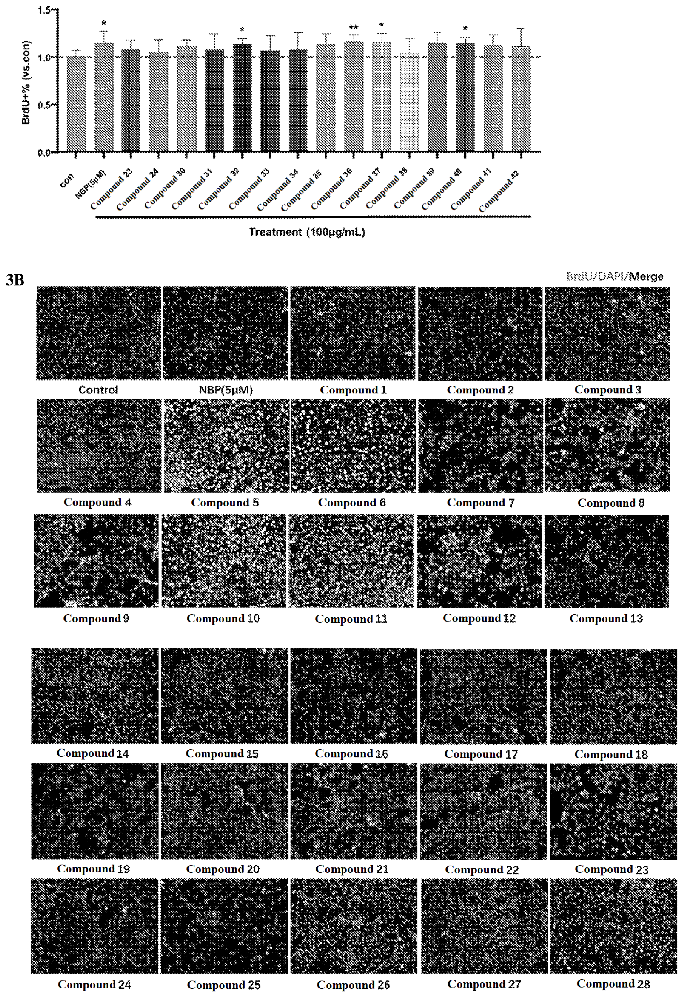

Compared with the blank control group, the polypeptide compounds 5-8, 10, 11, 15-20, 28, 32, 36, 37 and 40 of the invention can significantly promote the proliferation of C17.2 cells, as evidenced by a significant increase in the number of BrdU positive cells; in addition, compound 3 also showed a tendency to increase the number of BrdU positive cells (Fig. 3 A-B).

### Experimental result 2: The differentiation-promoting effect of the polypeptide of the present invention on primary neural stem cells (NSCs).

Images were acquired and statistically analyzed using a high-content cell imaging analysis system. The differentiation-promoting results of the polypeptide of the invention on primary neural stem cells (NSCs) are shown in Table 6:

**Table 6 The differentiation-promoting results of the polypeptide of the invention on primary neural stem cells (NSCs)**

| **The polypeptide of the invention** | **Average fluorescence intensity** | **Total neurite length** | **Longest neurite length** | **Number of neurite terminals** | Neurite **initiation points (branches)** |
|---|---|---|---|---|---|
| Compound 1 | ** | ** | *** | *** | *** |
| Compound 2 | / | ** | ** | ** | * |
| Compound 3 | * | ** | ** | * | * |
| Compound 4 | / | ** | *** | ** | ** |
| Compound 5 | / | ** | *** | * | *** |
| Compound 6 | / | * | * | / | * |
| Compound 7 | / | * | * | / | / |
| Compound 8 | / | / | / | / | / |
| Compound 9 | / | / | / | / | / |
| Compound 10 | / | * | / | ** | / |
| Compound 11 | / | *** | ** | ** | *** |
| Compound 12 | / | / | / | / | / |
| Compound 13 | / | / | / | / | / |
| Compound 14 | / | / | / | / | / |
| Compound 15 | / | / | * | / | / |
| Compound 16 | * | / | / | / | / |
| Compound 17 | / | / | / | / | / |
| Compound 18 | / | / | / | / | / |
| Compound 19 | / | / | / | / | / |
| Compound 20 | / | / | * | / | * |
| Compound 21 | / | / | / | / | / |
| Compound 22 | / | * | * | * | * |
| Compound 23 | / | * | / | * | / |
| Compound 24 | * | / | / | / | * |
| Compound 25 | * | / | / | / | / |
| Compound 26 | / | / | / | / | / |
| Compound 27 | / | * | ** | * | ** |
| Compound 28 | / | ** | / | * | / |
| Compound 29 | / | / | / | / | / |
| Compound 30 | *** | / | * | / | / |
| Compound 31 | / | / | / | / | / |
| Compound 32 | / | / | / | / | / |
| Compound 33 | / | / | / | / | / |
| Compound 34 | * | * | * | ** | * |
| Compound 35 | * | * | ** | * | * |
| Compound 36 | / | * | / | * | / |
| Compound 37 | / | / | / | / | / |
| Compound 38 | / | / | / | / | / |
| Compound 39 | / | / | / | / | / |
| Compound 40 | * | / | / | / | / |
| Compound 41 | / | * | / | / | * |
| Compound 42 | * | / | / | / | / |

Compared with the blank control group, *p < 0.05, **p < 0.01, ***p < 0.001; significant differences were observed in all 3 batches of experiments using the positive drug NGF; "/" indicates no significant difference, and a small number of samples showed cytotoxicity.

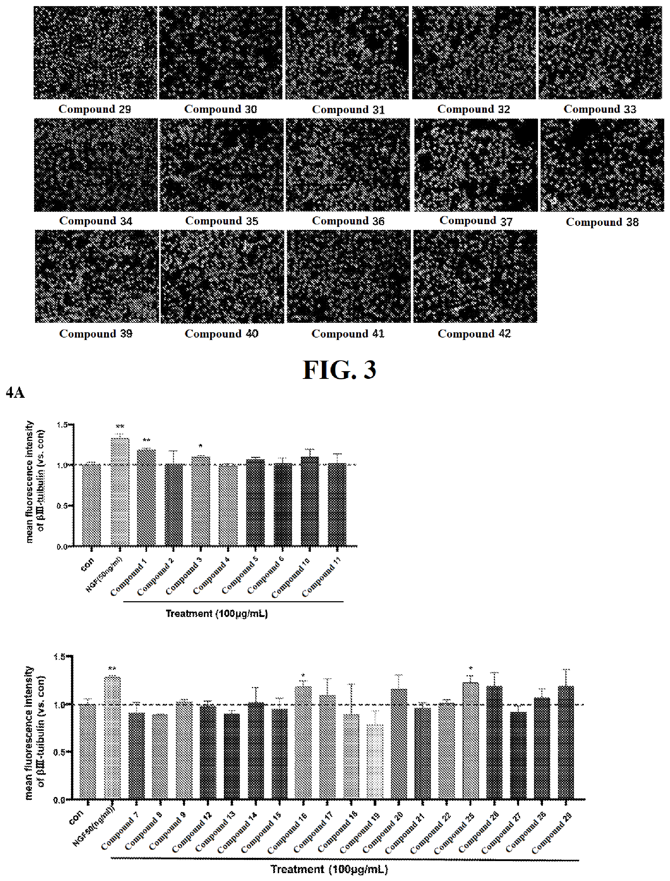

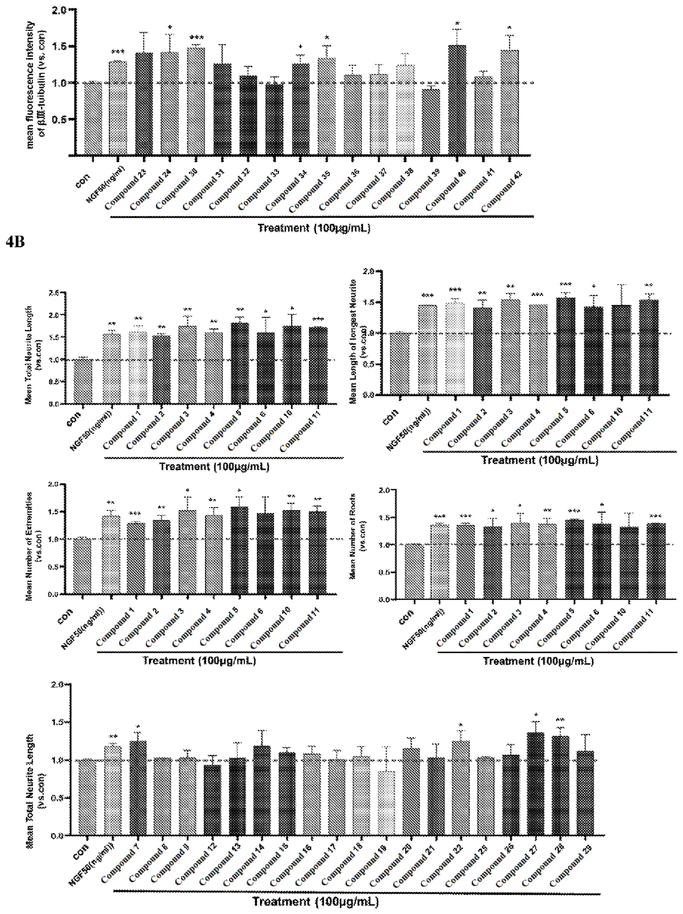

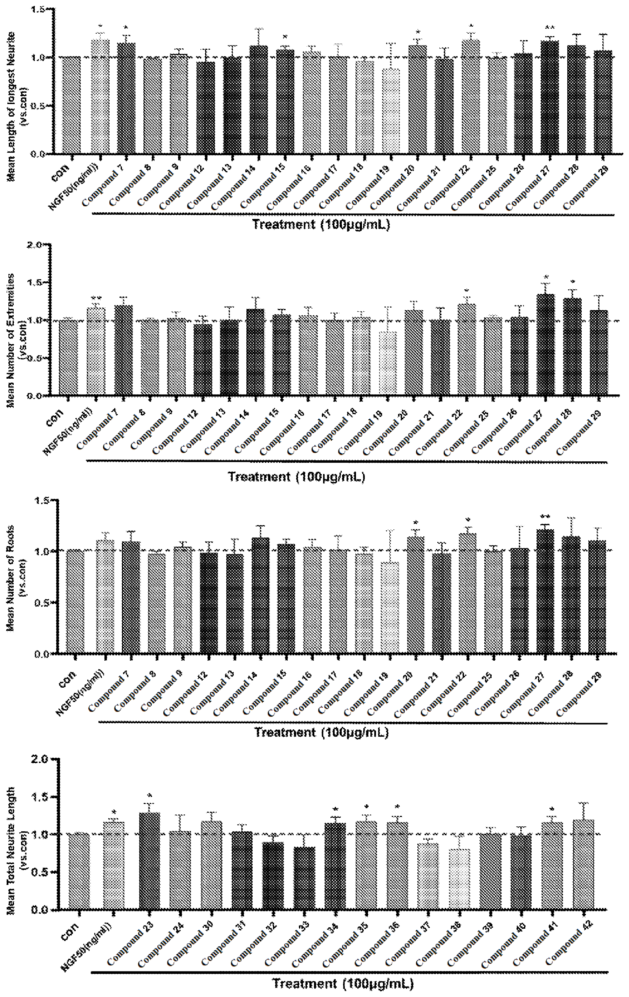

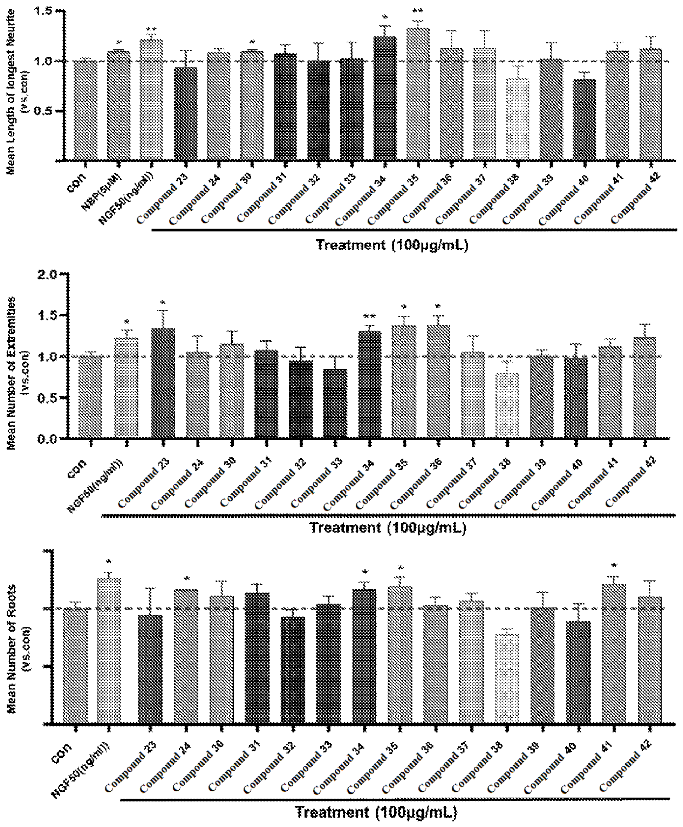

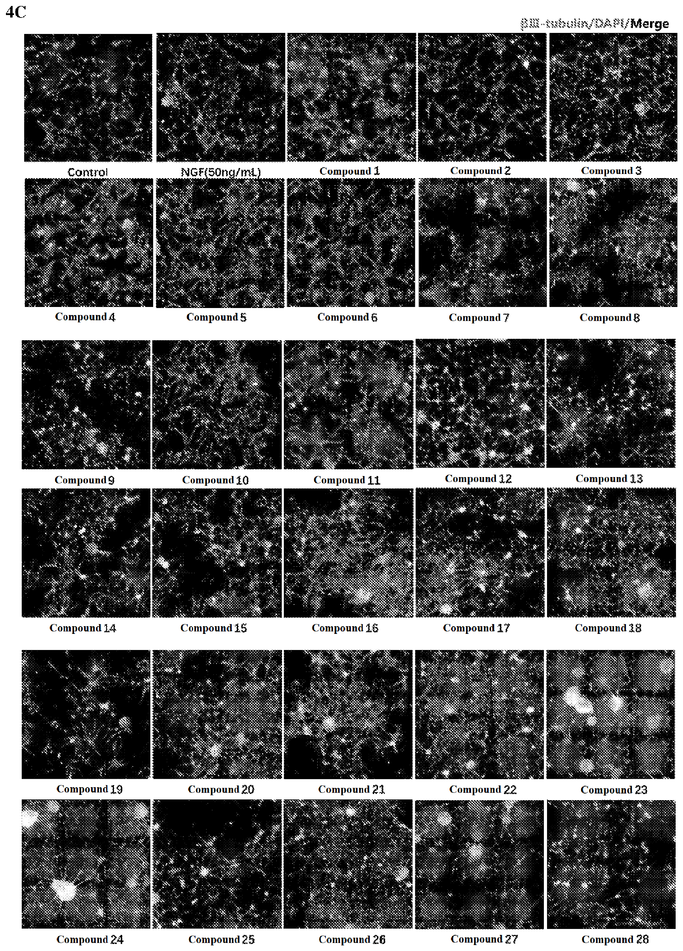

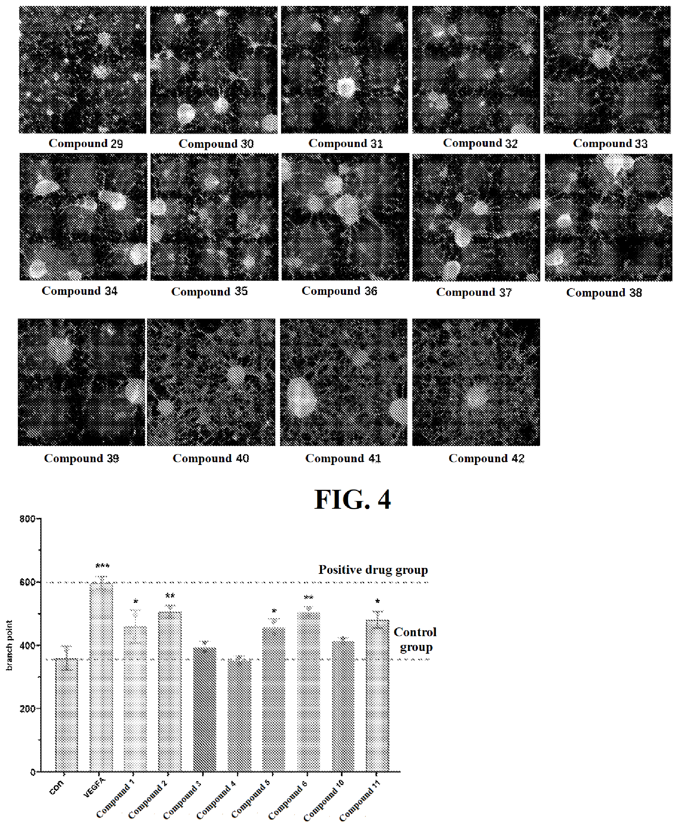

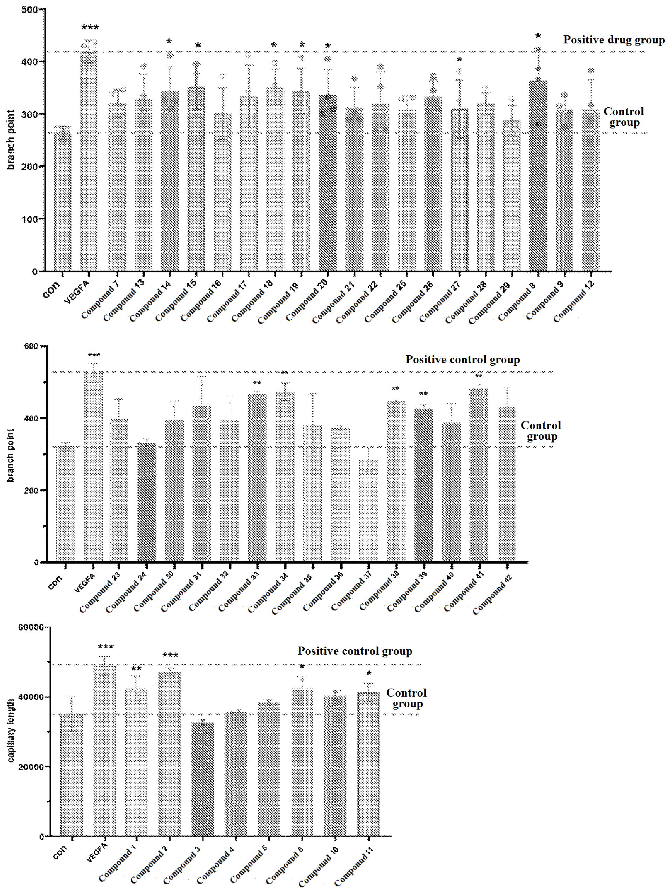

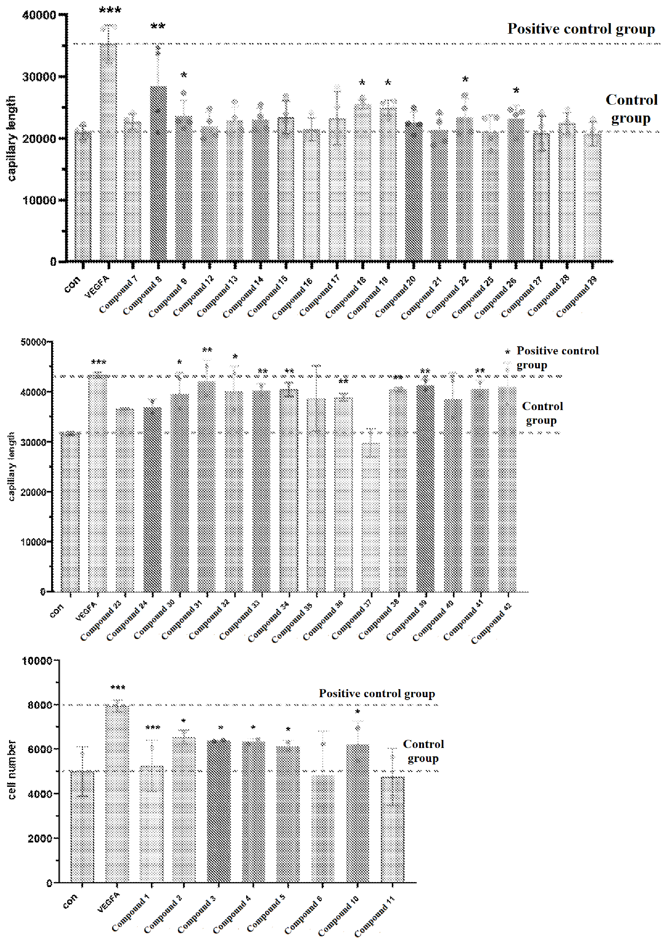

Compared with the blank control group, the polypeptide compounds 1, 3, 16, 24, 25, 30, 34, 35, 40 and 42 of the invention can promote the differentiation of NSCs into neuronal cells, as evidenced by a significant increase in the mean fluorescence intensity of the neuronal marker protein BIII-tubulin. The polypeptide compounds 10, 17, 20, 23, 26, 29, 31 and 38 of the present invention also show a tendency to enhance the mean fluorescence intensity of βIII-tubulin (Fig. 4A). In addition, the results of the Neurite Outgrowth Assay confirmed that the polypeptide compounds 1, 2-5, 11, 22, 27, 34 and 35 of the invention all promote neurite outgrowth, specifically manifested as increased total neurite length, increased maximum neurite length, increased number of neurite terminals, and increased number of neurite initiation points (branches). The polypeptide compounds 6, 7, 10, 15, 20, 23, 28, 30, 36 and 41 of the invention also show a tendency to promote neurite outgrowth in the four evaluation indices (Fig. 4B). Based on the results of the two analytical methods, it can be concluded that the polypeptide compounds 1, 3, 34 and 35 of the invention can promote the differentiation of NSCs into neuronal cells with stable effects.

### Example 4: Study on the angiogenesis-promoting effect of the polypeptide of the invention

First, mouse brain microvascular pellets were obtained by differential centrifugation. Brain microvascular endothelial cells (BMECs) and brain microvascular smooth muscle cells (BMVSMCs) were isolated by digestion with collagenase II, respectively, and cultured in a cell incubator with 5% CO₂ at 37°C . Cells at passages 3-6 in good growth condition and logarithmic growth phase were selected for the in vitro angiogenesis assay.

One night before the experiment, the high-concentration matrigel was transferred from a -20°C or -80°C refrigerator to a 4°C refrigerator and allowed to thaw slowly overnight in the 4°C refrigerator. Pipette tips required for the formal experiment were sterilized in advance and pre-cooled in a 4°C refrigerator for subsequent use.

On the day of the experiment, the matrigel was added to a 96-well plate 1 h in advance and placed in a 37°C cell incubator until it solidified. Subsequently, the cells were washed 2 times with 2 mL of PBS buffer, followed by digestion with 0.25% trypsin to obtain EC and VSMC pellets, respectively, and cell counting was performed. A uniform cell suspension containing 5×10³ BMVSMCs and 1×10³ BMECs per well was then seeded into the 96-well plate pre-coated with the matrigel, and cultured in a 37°C, 5% CO₂ cell incubator for 3 days.

During the culture period, tube formation was observed daily. The medium was changed on day 4, followed by treatment with the polypeptide of the invention at 0.1 mg/L or VEGFA at 10/15/20 ng/L for 48 h, respectively.

On day 5, the cell nuclei and live cells were labeled with Hoechst 33342 and Calcein AM, respectively. High-content brightfield and fluorescence imaging were then performed using a high-content screening system. Finally, angiogenesis in each treatment group was analyzed using the Angiogenesis Analyzer plugin for ImageJ, and statistical analysis was carried out with GraphPad Prism 10.

Experimental results: The experimental results of the polypeptides of the invention in promoting angiogenesis are shown in Table 7:

**Table 7 The experimental results of the polypeptides of the invention in promoting angiogenesis**

| **The polypeptide of the invention** | **Number of intersections** | **Branch length** | **Cell count** |
|---|---|---|---|
| Compound 1 | * | ** | *** |
| Compound 2 | ** | *** | * |
| Compound 3 | / | / | * |
| Compound 4 | / | / | * |
| Compound 5 | * | / | * |
| Compound 6 | ** | * | / |
| Compound 7 | / | / | ** |
| Compound 8 | * | ** | / |
| Compound 9 | / | * | / |
| Compound 10 | / | / | * |
| Compound 11 | * | * | / |
| Compound 12 | / | / | / |
| Compound 13 | / | / | / |
| Compound 14 | * | / | * |
| Compound 15 | * | / | / |
| Compound 16 | / | / | / |
| Compound 17 | / | / | ** |
| Compound 18 | * | * | * |
| Compound 19 | * | * | / |
| Compound 20 | * | / | / |
| Compound 21 | / | / | / |
| Compound 22 | / | * | / |
| Compound 23 | / | / | / |
| Compound 24 | / | / | / |
| Compound 25 | / | / | ** |
| Compound 26 | * | * | / |
| Compound 27 | / | / | / |
| Compound 28 | / | / | / |
| Compound 29 | / | / | / |
| Compound 30 | / | * | ** |
| Compound 31 | / | ** | / |
| Compound 32 | / | * | ** |
| Compound 33 | ** | ** | ** |
| Compound 34 | ** | ** | ** |
| Compound 35 | / | / | ** |
| Compound 36 | / | ** | ** |
| Compound 37 | / | / | / |
| Compound 38 | ** | ** | * |
| Compound 39 | ** | ** | * |
| Compound 40 | / | / | * |
| Compound 41 | ** | ** | *** |
| Compound 42 | / | * | * |

Compared with the blank control group, *p < 0.05, **p < 0.01, ***p < 0.001; significant differences were observed in all 3 batches of experiments using the positive drug VEGFA; "/" indicates no significant difference, and some of the samples showed cytotoxicity.

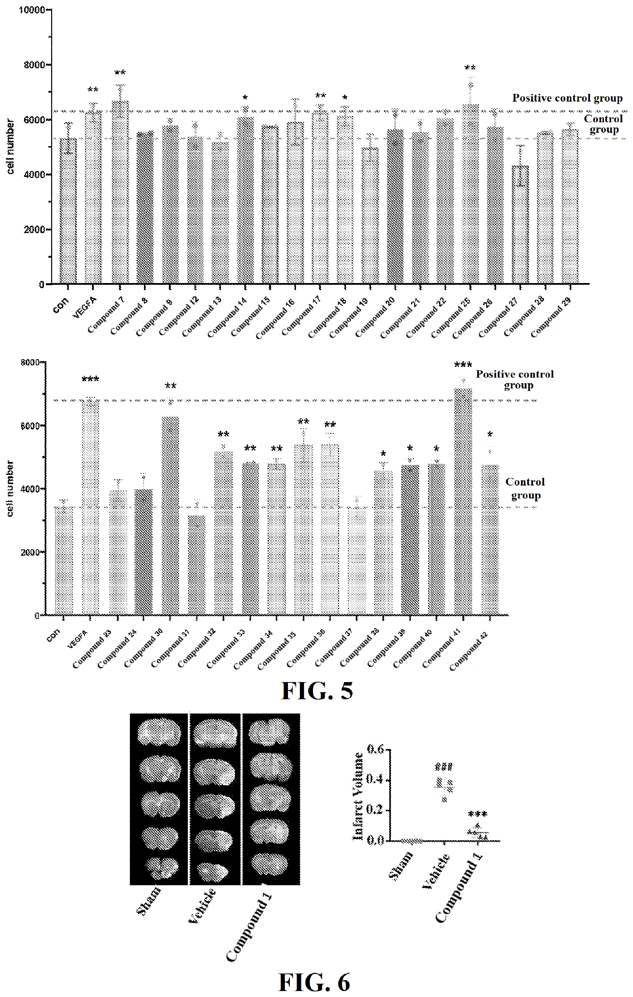

Compared with the normal group, the polypeptide compounds 1, 2, 6, 8, 11, 18, 19, 26, 33, 34, 38, 39 and 41 of the invention exhibited angiogenesis-promoting effects; the polypeptide compounds 5, 14, 15 and 20 of the invention showed promoting effects on the number of junctions but no significant effects on branch length, the polypeptide compounds 9, 22, 30-32, 36 and 42 of the invention exerted promoting effects on branch length but no significant effects on the number of junctions, and the polypeptide compounds 3, 4, 7, 10, 17, 25, 35 and 40 of the invention only promoted cell proliferation. Overall, they did not have significant angiogenesis-promoting effects (Fig. 5).

### Example 5: Effect of the polypeptide of the invention on cerebral infarction volume in rats with stroke

Model establishment: Male SD rats, weighing 230-260g.

A rat model of transient middle cerebral artery occlusion (tMCAO) was established using the intraluminal filament technique. After anesthesia, a male SD rat was fixed on the operating board in a supine position. The neck was shaved with a razor, disinfected with iodophor, and the superficial fascia was incised slightly to the left of the midline of the neck. The left common carotid artery (CCA), internal carotid artery (ICA) and external carotid artery (ECA) were isolated, taking care not to injure the vagus nerve. The CCA and ECA were ligated with 6-0 surgical sutures, and the ICA was temporarily occluded with a microvascular clip. An incision was made in the ECA using a sterile needle. A 0.36 mm nylon filament was inserted into the ICA and gently advanced, keeping it parallel to the internal carotid artery as much as possible, until slight resistance was felt. The loose ligature on the ICA and the microvascular clip were released. A ligation was performed at the ECA incision to secure the filament and block blood reflux from the internal carotid artery. The subcutaneous tissue and skin were sutured. After wiping away blood with sterile cotton balls, iodophor was applied for disinfection. After 2 h of occlusion, the nylon filament was slowly withdrawn to establish the cerebral ischemia-reperfusion injury model. Body temperature of the animals was maintained at 37 ± 1°C during and after the operation until they fully recovered consciousness and resumed spontaneous activity.

Success criteria for modeling: (1) diminished or absent withdrawal response to painful stimulation of the left limbs; (2) inability to extend the left forelimb downward when the tail was lifted and suspended; (3) circling or leaning to the left during ambulation.

Grouping and administration: Polypeptide compound 1 of the invention (10 mg/kg): prepared with the polypeptide and normal saline, filtered through a 0.22 µm filter membrane, and stored for later use. SD rats were randomly divided into the following groups: Sham group: animals received sham surgery and an equal volume of normal saline; Vehicle group: animals received tMCAO and an equal volume of normal saline; Treatment group: animals received tMCAO and were injected with polypeptide compound 1 of the invention via the tail vein.

### Experimental method:

TTC (2,3,5-triphenyltetrazolium chloride) is the most commonly used dye for ischemic infarction. It reacts with dehydrogenases to appear red (normal tissue) or pale (ischemic tissue).

Cerebral infarct volume was determined by TTC staining. On day 7 after surgery, rats were anesthetized and decapitated for brain extraction, 5 consecutive 2-mm-thick coronal brain slices were cut at equal intervals starting from the frontal pole, and incubated in 0.5% TTC staining solution at 37°C for 30 min in the dark, with the brain slices turned over every 15 min. After uniform staining, the slices were fixed in 4% paraformaldehyde solution for at least 12 h and stored in a 4°C refrigerator. The white areas in the brain tissue represented cortical infarction.

Infarct area calculation: Ischemic volume ratio = (sum of white ischemic areas of all slices) / (sum of total brain areas of all slices) * 100%. The result is shown in Fig. 6.

**Experimental results:** Fig. 6 shows that, compared with the model group, the cerebral infarction volume in the group administered with the polypeptide of the invention was significantly reduced.

### Example 6: Effects of the polypeptide of the invention on pathological damage to the hippocampus and cortex of rats with stroke

Experimental animals, model establishment, grouping and drug administration are the same as in Example 5.

### Experimental method:

Rats were deeply anesthetized and brains were harvested by transcardiac perfusion with 4% paraformaldehyde. The perfusion procedure was as follows: a rat was fixed in the supine position, an incision was made along the abdominal midline to expose the heart, then a perfusion needle was inserted into the right ventricle from the left ventricular apex along the direction of the aortic opening (about 3-5 mm), the right atrial appendage was cut open, and first perfused with heparinized phosphate-buffered saline (PBS) until the rat liver turned white and the effluent became blood-free, then 4% paraformaldehyde solution was used for perfusion until the rat's body became rigid, and subsequently the rat was decapitated and the brain was removed. The removed brains were embedded in paraffin and sectioned into 5-8 µm thick slices. After deparaffinization, the slices were stained with eosin staining solution, and then mounted with neutral resin. The mounted slices were observed under an upright microscope for the cellular morphology of the hippocampal tissue on the injured side, and images were collected.

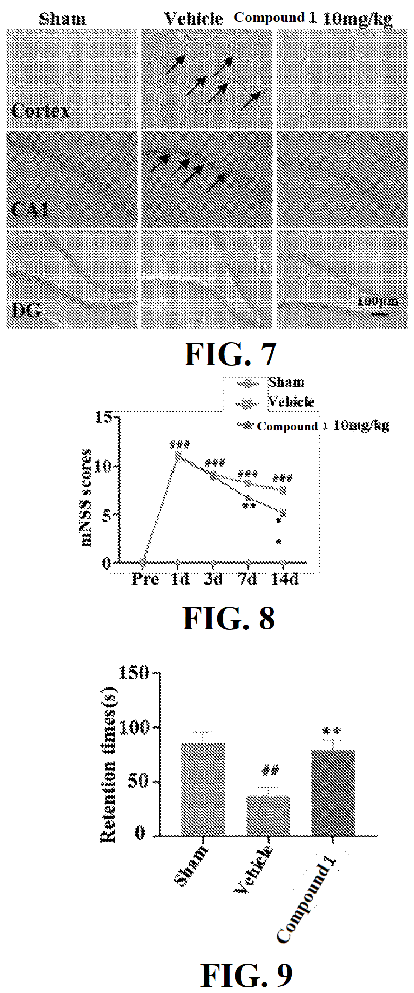

**Experimental results:** As shown in Fig. 7 (black arrows indicate injured cells), on the 14th day after model establishment, the model group exhibited more cellular injury in the hippocampus and cortex compared with the treatment group, including nuclear pyknosis, intensified staining, and damaged cellular morphology, indicating that the polypeptide of the invention can alleviate injury and death of hippocampal and cortical cells in rats with stroke.

### Example 7: Effects of the polypeptide of the invention on neuromotor function in rats with stroke

Experimental animals, model establishment, grouping and drug administration are the same as in Example 5.

**Experimental method:** After model establishment, drugs were administered via the tail vein. Neurological function was assessed using the modified Neurological Severity Score (mNSS) on days 1, 7, and 14 post-administration.

**Experimental results:** As shown in Fig. 8, compared with the model control group, the mNSS scores of rats in the polypeptide group of the invention were significantly decreased on days 7 and 14, indicating that the neurological dysfunctions of rats, including sensation, reflex, motor and balance functions, were all improved. As shown in Fig. 9, the rotarod test showed that the polypeptide group of the invention spent a significantly longer time on the rotarod compared with the model group, indicating that the balance ability and endurance were restored. All the above results demonstrate that the polypeptide of the invention can significantly improve the neurological motor dysfunction in the late stage of cerebral ischemia-reperfusion in rats.

### Example 8: Effect of the polypeptide of the invention on hippocampal cell proliferation in rats with cerebral ischemia-reperfusion injury

Experimental animals, model establishment, grouping and drug administration are the same as in Example 5.

### Experimental method:

5-Bromodeoxyuridine (BrdU) was administered twice daily. After treatment, rat brains were harvested via cardiac perfusion. The brain tissue was fixed in 4% paraformaldehyde (PFA), then dehydrated in 20% and 30% sucrose solutions successively until it sank to the bottom. After dehydration, the liquid on the tissue surface was carefully wiped off, and the tissue was embedded. Frozen sections were prepared at a thickness of 10 µm. The frozen sections were then subjected to BrdU immunofluorescence staining.

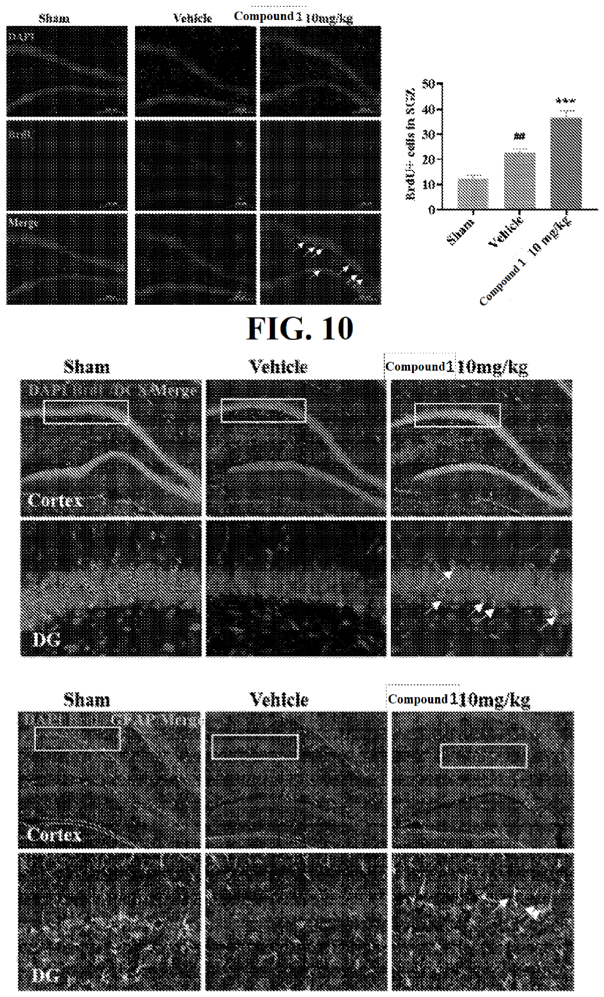

**Experimental results:** As shown in Fig. 10 (arrows indicate newborn cells), compared with the model group, the number of BrdU-positive cells in the SGZ region was significantly increased in the polypeptide group of the invention, indicating that the polypeptide of the invention can increase the number of newborn cells in the DG region of rats after stroke.

### Example 9: Effect of the polypeptide of the invention on the differentiation of hippocampal neural stem cells in rats with cerebral ischemia-reperfusion injury

Experimental animals, model establishment, grouping and drug administration are the same as in Example 5.

### Experimental method:

Double immunofluorescence staining for DCX (marker protein of immature neurons)/BrdU and GFAP (marker protein of astrocytes)/BrdU.

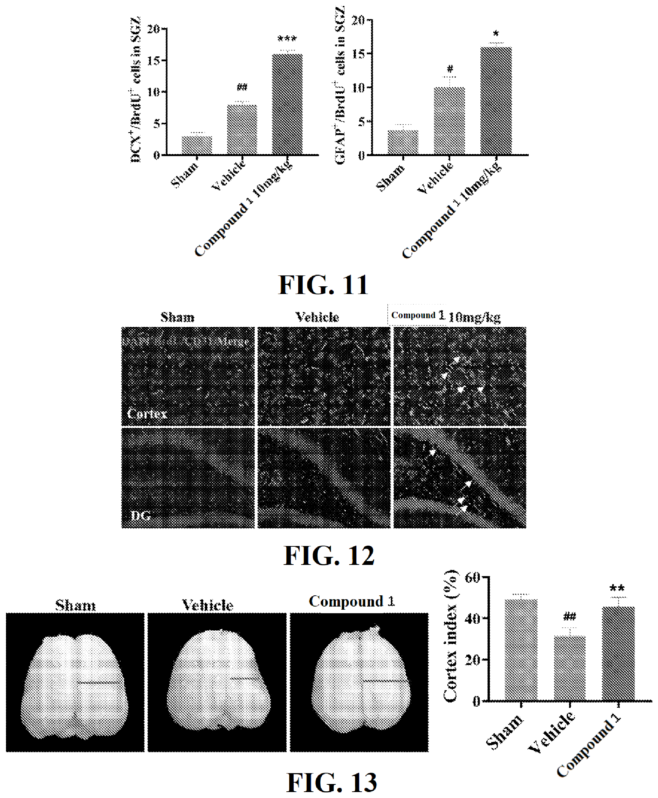

**Experimental Results:** As shown in Fig. 11 (arrows indicate newborn neurons and astrocytes), compared with the model group, the numbers of DCX/BrdU and GFAP/BrdU double-positive cells in the SGZ region were significantly increased in the polypeptide drug group, indicating that the numbers of newborn neurons and astrocytes were both elevated. It suggests that the polypeptide of the invention can promote the differentiation of neural stem cells into neurons and astrocytes after stroke, and has the effect of promoting neural stem cell differentiation.

### Example 10: Effect of the polypeptide of the invention on the expression of CD31, a vascular marker protein in the hippocampus of rats with cerebral ischemia

Experimental animals, model establishment, grouping and drug administration are the same as in Example 5.

### Experimental method:

Frozen sections of brain tissue from rats with cerebral ischemia-reperfusion injury were subjected to CD31 (vascular marker protein)/BrdU immunofluorescence double staining.

**Experimental results:** As shown in Fig. 12 (arrows indicate newborn blood vessels), compared with the model group, the number of CD31/BrdU double-positive cells in the SGZ region and the cortical region was significantly increased in the invention polypeptide group, indicating that the number of newborn blood vessels was significantly increased, suggesting that the invention polypeptide can promote angiogenesis in stroke rats.

### Example 11: Effects of the polypeptide of the invention on the expression of PSD95 and SYP in the hippocampus and cortex of rats with cerebral ischemia

Experimental animals, model establishment, grouping and drug administration are the same as in Example 5.

### Experimental method:

The cortical index was calculated for each group of rats 14 days after ischemia-reperfusion injury. tMCAO rats were treated with the polypeptide compound 1 of the invention for 14 days, and the expression of PSD95 and SYP was detected by Western blot.

**Experimental result 1:** The results showed that 14 days of ischemia-reperfusion injury caused significant atrophy and collapse of the rat cortical tissue (*P*<0.01). After treatment with the polypeptide compound 1 of the invention for 14 days, the cortical index was significantly increased (*P*<0.01), and the cortical atrophy on the injured side was significantly ameliorated (Fig. 13). These data indicate that the polypeptide compound 1 of the invention can significantly ameliorate the pathological tissue damage and alleviate cortical tissue atrophy in tMCAO rats.

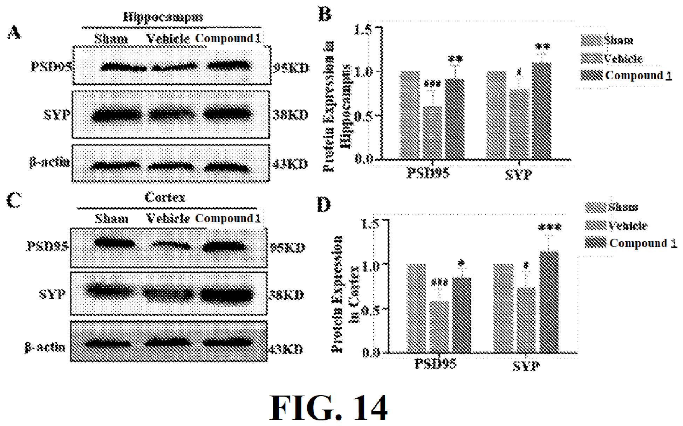

**Experimental result 2:** As shown in Fig. 14, compared with the Sham group, the expression of PSD95 and SYP in the hippocampus and cortex of rats in the Vehicle group was significantly decreased (*P*<0.05), while treatment with the polypeptide compound 1 of the invention significantly increased the expression of PSD95 and SYP (*P*<0.05). These results indicate that cerebral ischemia-reperfusion injury inhibits the expression of synaptic proteins in the hippocampus of SD rats and affects the formation of synaptic structures, which may be an important cause of neurological deficits. After treatment with the polypeptide compound 1 of the invention, the expression of synaptic proteins in the hippocampus of tMCAO rats was significantly increased. This suggests that the polypeptide compound 1 of the invention may restore neurological function in rats through synaptic remodeling.

In summary, the polypeptide of the invention can promote the proliferation of the neural stem cell line C17.2 and the differentiation of primary neural stem cells (NSCs), as well as promote angiogenesis in an in vitro cerebrovascular-like model. Meanwhile, it can effectively improve neurological motor dysfunction in stroke rats, significantly reduce cerebral infarct volume, and alleviate pathological damage in the hippocampus and cortex, such as reducing injury and death of hippocampal and cortical cells in stroke rats. Furthermore, the polypeptide of the invention also promotes the proliferation and differentiation of neural stem cells in stroke rats, increases the number of hippocampal neurons, glial cells and new blood vessels during stroke recovery, enhances neurogenesis and angiogenesis in stroke rats, and elevates the expression of synaptic proteins in the hippocampus, thereby restoring neurological function in rats through synaptic remodeling. This indicates that the polypeptide of the invention has good efficacy in treating stroke.

Although the present invention discloses the above Examples, the embodiments of the invention are not limited to the above Examples, and any other changes, modifications, replacements, combinations, and simplifications that do not deviate from the invention should be considered as equivalents and are included in the scope of protection of the invention.

## Claims

1. A polypeptide or a physiologically compatible salt thereof, wherein the amino acid sequence of the polypeptide is selected from:
H-Ala-Leu-Pro-Ala-Pro-Gly-Thr-Leu-OH (SEQ ID No. 1);
H*-D-*Ala-Leu-Pro-Ala-Pro-Gly-Thr-Leu-OH (SEQ ID No. 2);
H-Ala*-D-*Leu-Pro-Ala-Pro-Gly-Thr-Leu-OH (SEQ ID No. 3);
H-Ala-Leu*-D-*Pro-Ala-Pro-Gly-Thr-Leu-OH (SEQ ID No. 4);
H-Ala-Leu-Pro*-D-*Ala-Pro-Gly-Thr-Leu-OH (SEQ ID No. 5);
H-Ala-Leu-Pro-Ala*-D-*Pro-Gly-Thr-Leu-OH (SEQ ID No. 6);
H-Ala-Leu-Pro-Ala-Pro-Gly*-D-*Thr-Leu-OH (SEQ ID No. 7);
H-Ala-Leu-Pro-Ala-Pro-Gly-Thr*-D-*Leu-OH (SEQ ID No. 8);
Ac-Ala-Leu-Pro-Ala-Pro-Gly-Thr-Leu-OH (SEQ ID No. 9);
H-Ala-Leu-Pro-Ala-Pro-Gly-Thr-Leu-NH2 (SEQ ID No. 10);
Ac-Ala-Leu-Pro-Ala-Pro-Gly-Thr-Leu-NH2 (SEQ ID No. 11);
H-Ala-Leu-Pro-Ala-Pro-Gly-Thr-Ala-OH (SEQ ID No. 12);
H-Ala-Leu-Ala-Ala-Pro-Gly-Thr-Leu-OH (SEQ ID No. 13);
H-Ala-Leu-Gly-Ala-Pro-Gly-Thr-Leu-OH (SEQ ID No. 14);
H-Ala-Leu-Pro-Ala-Gly-Gly-Thr-Leu-OH (SEQ ID No. 15);
H-Ala-Ala-Pro-Ala-Pro-Gly-Thr-Leu-OH (SEQ ID No. 16);
H-Ala-Leu-Pro-Ala-Ala-Gly-Thr-Leu-OH (SEQ ID No. 17);
H-Ala-Leu-Pro-Ala-Pro-Gly-Ala-Leu-OH (SEQ ID No. 18);
H-Leu-Ala-Pro-Ala-Pro-Gly-Thr-Leu-OH (SEQ ID No. 19);
H-Leu-Ala-Pro-Ala-Pro-Gly-Thr-Ile-OH (SEQ ID No. 20);
H-Ala-Leu-Pro-Ala-Pro-Gly-Thr-Ile-OH (SEQ ID No. 21);
H-Gln-Leu-Pro-Ala-Pro-Gly-Thr-Leu-OH (SEQ ID No. 22);
H-Pro-Ile-Ala-Ser-Gln-His-Leu-Asn-Leu-Ala-Pro-Ala-Pro-Gly-Thr-Ile-OH (SEQ ID No. 23);
H-Ala-Lys-Asn-Gln-Leu-Pro-Ala-Pro-Gly-Thr-Leu-Gln-His-Phe-Cys-OH (SEQ ID No. 24);
H-Gln-Leu-Pro-Ala-Pro-Gly-Thr-Leu-Gln-His-Phe-OH (SEQ ID No. 25);
H-Pro-Ala-Pro-Gly-Thr-Leu-OH (SEQ ID No. 26);
H-Pro-Ala-Pro-Gly-Thr-OH (SEQ ID No. 27);
H-Pro-Gly-Thr-Leu-OH (SEQ ID No. 28);
H-Leu-Pro-Ala-Pro-Gly-Thr-Leu-OH (SEQ ID No. 29);
H-Ala-Leu-Pro-Ala-Pro-OH (SEQ ID No. 30);
H-Leu-Pro-Ala-Pro-OH (SEQ ID No. 31);
H-Leu-Pro-Ala-Pro-Gly-Thr-OH (SEQ ID No. 32);
H-Leu-Pro-Ala-Pro-Gly-OH (SEQ ID No. 33);
H-Ala-Pro-Gly-Thr-Leu-OH (SEQ ID No. 34);
H-Ala-Leu-Pro-Ala-OH (SEQ ID No. 35);
H-Pro-Ala-Pro-Gly-OH (SEQ ID No. 36);
H-Ala-Pro-Gly-Thr-OH (SEQ ID No. 37);
H-Gly-Thr-Leu-OH (SEQ ID No. 38);
H-Ala-Leu-Pro-Ala-Pro-Gly-Thr-OH (SEQ ID No. 39);
H-Ala-Leu-Pro-Ala-Pro-Gly-Oh (SEQ ID No. 40);
Ac-Ala-Leu-Pro-Ala*-D-*Pro-Gly-Thr-Leu-NH2 (SEQ ID No. 41); and
Ac-Ala-Leu-Pro*-D-*Ala-Pro-Gly-Thr-Leu-NH2 (SEQ ID No. 42).

2. The polypeptide of claim 1, wherein the polypeptide is selected from: compound 1, compound 2, compound 3, compound 4, compound 5, compound 6, compound 7, compound 8, compound 10, compound 11, compound 15, compound 16, compound 17, compound 18, compound 19, compound 20, compound 22, compound 23, compound 24, compound 25, compound 26, compound 27, compound 28, compound 29, compound 30, compound 31, compound 32, compound 33, compound 34, compound 35, compound 36, compound 37, compound 38, compound 39, compound 40, compound 41, compound 42, or physiologically compatible salts thereof.

3. Use of the polypeptide of claim 1 or 2 or a physiologically compatible salt thereof in the preparation of a drug for treating or preventing neurological injury-related diseases.

4. The use of claim 3, wherein the neurological injury-related disease involves abnormal cerebral microvascular angiogenesis.

5. The use of claim 4, wherein the neurological injury-related disease includes stroke, diabetic neuropathy, brain injury, neurodegenerative diseases, vascular dementia, cerebrovascular diseases, and epilepsy.

6. The use of claim 5, wherein the neurodegenerative disease includes Alzheimer's disease, Parkinson's disease, amyotrophic lateral sclerosis, Huntington's disease, multiple sclerosis, and HIV-1-related dementia.

7. The use of claim 5, wherein the brain injury includes chronic traumatic encephalopathy and cerebral ischemia-reperfusion injury.

8. The use of claim 5, wherein the stroke includes ischemic stroke, hemorrhagic stroke, and stroke sequelae.

9. The use of claim 8, wherein the polypeptide improves neurological dysfunction in stroke.

10. The use of claim 8, wherein the polypeptide promotes the number of hippocampal neurons, glial cells, and new blood vessels in the later stage of stroke.

11. The use of claim 8, wherein the polypeptide promotes neurogenesis and angiogenesis in stroke.

12. The use of claim 8, wherein the stroke sequelae include limb dysfunction, mental and intellectual impairment, speech dysfunction, and dysphagia.

13. A composition, comprising the polypeptide of claim 1 or 2 or a physiologically compatible salt thereof and a physiologically compatible excipient.

14. The composition of claim 13, wherein the composition is a pharmaceutical composition in any form selected from the group consisting of oral liquid, tablet, granule, capsule, dripping pill, injection, transdermal preparation, topical lotion, and implantable preparation.

15. The composition of claim 13, wherein the composition is a health care product composition.
